# EUROPEAN PATENT SPECIFICATION

(11) **EP 1 260 590 B1**
(45) Date of publication and mention of the grant of the patent: **14.02.2007**
(21) Application number: 01908140.5
(22) Date of filing: 28.02.2001
(51) Int. Cl.: C12Q 1/02, C12N 15/09, C12N 1/21, G01N 33/15, G01N 33/50, A61K 45/00, A61P 31/04, A61P 33/06, A61P 43/00

(54) **METHOD OF SCREENING SUBSTANCE SPECIFICALLY INHIBITING NON-MEVALONATE PATHWAY**
VERFAHREN ZUM SCREENING NACH SUBSTANZEN, DIE SPEZIFISCH DEN "NICHT-MEVALONAT"-PFAD INHIBIEREN
PROCEDE DE CRIBLAGE DE SUBSTANCE A INHIBITION SPECIFIQUE DE LA VOIE NON MEVALONATE

(30) Priority: 02.03.2000 JP 2000056753
(43) Date of publication of application: 27.11.2002
(73) Proprietor: TOUDAI TLO, Ltd., Tokyo 113-0033 (JP)
(72) Inventor: SETO, Haruo, Hachioji-shi, Tokyo 192-0902 (JP); KUZUYAMA, Tomohisa, Tokyo 155-0032 (JP); MITSUI, Nobuo, Iida-shi, Nagano 395-0806 (JP)
(74) Representative: polypatent
(86) International application number: PCT/JP2001/001501
(87) International publication number: WO 2001/064943

(56) References cited:
- WO-A-00/01650
- DAIRI T ET AL: "Cloning of the gene encoding 3-hydroxy-3-methylglutaryl coenzyme A reductase from terpenoid antibiotic-producing Streptomyces strains." MOLECULAR & GENERAL GENETICS : MGG. JAN 2000, vol. 262, no. 6, January 2000 (2000-01), pages 957-964, XP002303427 ISSN: 0026-8925
- ROHMER M ED - JUCKER ET AL: "ISOPRENOID BIOSYNTHESIS VIA THE MEVALONATE-INDEPENDENT ROUTE, A NOVEL TARGET FOR ANTIBACTERIAL DRUGS?" PROGRESS IN DRUG RESEARCH, BASEL : BIRKHAEUSER, CH, vol. VOL. 50, 1998, pages 135-154, XP000906878 ISBN: 3-7643-5821-1
- HAHN F M ET AL: "Escherichia coli open reading frame 696 is idi, a nonessential gene encoding isopentenyl diphosphate isomerase" JOURNAL OF BACTERIOLOGY, WASHINGTON, DC, US, vol. 181, no. 15, August 1999 (1999-08), pages 4499-4504, XP002237077 ISSN: 0021-9193
- SETO H ET AL: "Simultaneous Operation of the Mevalonate and Non-mevalonate Pathways in the Biosynthesis of Isopentenyl diphosphate in Streptomyces aeriouvifer" TETRAHEDRON LETTERS, ELSEVIER SCIENCE PUBLISHERS, AMSTERDAM, NL, vol. 37, no. 44, 28 October 1996 (1996-10-28), pages 7979-7982, XP004031021 ISSN: 0040-4039
- TAKAHASHI S ET AL: "PURIFICATION, CHARACTERIZATION, AND CLONING OF A EUBACTERIAL 3-HYDROXY-3-METHYLGLUTARYL COENZYME A REDUCTASE, A KEY ENZYME INVOLVED IN BIOSYNTHESIS OF TERPENOIDS" JOURNAL OF BACTERIOLOGY, WASHINGTON, DC, US, vol. 181, no. 4, February 1999 (1999-02), pages 1256-1263, XP002937866 ISSN: 0021-9193
- KANEDA K ET AL: "AN UNUSUAL ISOPENTENYL DIPHOSPHATE ISOMERASE FOUND IN THE MEVALONATE PATHWAY GENE CLUSTER FROM STREPTOMYCES SP. STRAIN CL190" PROCEEDINGS OF THE NATIONAL ACADEMY OF SCIENCES OF USA, NATIONAL ACADEMY OF SCIENCE. WASHINGTON, US, vol. 98, no. 3, 30 January 2001 (2001-01-30), pages 932-937, XP002947190 ISSN: 0027-8424
- TAKAGI M ET AL: "A GENE CLUSTER FOR THE MEVALONATE PATHWAY FROM STREPTOMYCES SP. STRAIN CL190" JOURNAL OF BACTERIOLOGY, WASHINGTON, DC, US, vol. 182, no. 15, August 2000 (2000-08), pages 4153-4157, XP002937865 ISSN: 0021-9193
- KUZUYAMA T. ET AL.: 'Fosmidomycin, a specific inhibitor of ... in the nonmevalonate pathway for ...' TETRAHEDRON LETTERS vol. 39, no. 43, 1998, pages 7913 - 7916, XP002942010
- TOMOYUKI KUZUYAMA ET AL.: 'Minogasarete ita isoprenoid seigousei keiro: Hi mevalonic san keiro to sono tokuiteki sogaizai' NIPPON YUKA GAKKAI vol. 49, no. 2, 20 February 2000, pages 119 - 125, XP002942011
- HARUO SETO: 'Atarashii yakuzai hyouteki: Minogasarete ita hi mevalonic san keiro' BIOSCIENCE TO INDUSTRY vol. 57, no. 7, 1999, pages 449 - 453, XP002942012
- HASSAN JOMAA ET AL.: 'Inhibitors of the nonmevalonate pathway of isoprenoid biosynthesis as antimalarial drugs' SCIENCE vol. 285, 1999, pages 1573 - 1576, XP002942013

### SEQUENCE LISTING

<110> SET0 Haruo and KUZUYAMA Tomohisa
<120> A method for screening a substance which specifically inhibits non-mevalonate pathway
<130> A11029A
<160> 13
<210> 1
   <211> 40
   <212> DNA
   <213> Artificial Sequence
<220>
   <223> Description of Artificial Sequence: an artificially synthesized primer sequence
<400> 1
   ggaagcttgg aggttgtttt atgcagaaaa gacaaaggga 40
<210> 2
   <211> 28
   <212> DNA
   <213> Artificial Sequence
<220>
   <223> Description of Artificial Sequence: an artificially synthesized primer sequence
<400> 2
   ggtctagatc actgcgcatg gttgtcga 28
<210> 3
   <211> 40
   <212> DNA
   <213> Artificial Sequence
<220>
   <223> Description of Artificial Sequence: an artificially synthesized primer sequence
<400> 3
   ggtctagagg aggttgtttt atgcgcagtg aacacccgac 40
<210> 4
   <211> 28
   <212> DNA
   <213> Artificial Sequence
<220>
   <223> Description of Artificial Sequence: an artificially synthesized primer sequence
<400> 4
   ggagatcttc atgcgccctc gctcagca 28
<210> 5
   <211> 40
   <212> DNA
   <213> Artificial Sequence
<220>
   <223> Description of Artificial Sequence: an artificially synthesized primer sequence
<400> 5
   ggagatctgg aggttgtttt atgacgacag gtcagcgcac 40
<210> 6
   <211> 28
   <212> DNA
   <213> Artificial Sequence
<220>
   <223> Description of Artificial Sequence: an artificially synthesized primer sequence
<400> 6
   ggatcgattt agatcccttc cagggcag 28
<210> 7
   <211> 40
   <212> DNA
   <213> Artificial Sequence
<220>
   <223> Description of Artificial Sequence: an artificially synthesized primer sequence
<400> 7
   ggatcgatgg aggttgtttt atgaccagcg cccaacgcaa 40
<210> 8
   <211> 28
   <212> DNA
   <213> Artificial Sequence
<220>
   <223> Description of Artificial Sequence: an artificially synthesized primer sequence
<400> 8
   gggcatgctc atcgtgtgct tcccgtcg 28
<210> 9
   <211> 1050
   <212> DNA
   <213> Streptomyces
<400> 9
   ggaggttgtt ttatgcagaa aagacaaagg gagctgagtg cgttgacact accgacctcg 60
   gctgaggggg tatcagaaag ccaccgggcc cgctcggtcg gcatcggtcg cgcccacgcc 120
   aaggccatcc tgctgggaga gcatgcggtc gtctacggag cgccggcact cgctctgccg 180
   attccgcagc tcacggtcac ggccagcgtc ggctggtcgt ccgaggcctc cgacagtgcg 240
   ggtggcctgt cctacacgat gaccggtacg ccgtcgcggg cactggtgac gcaggcctcc 300
   gacggcctgc accggctcac cgcggaattc atggcgcgga tgggcgtgac gaacgcgccg 360
   cacctcgacg tgatcctgga cggcgcgatc ccgcacggcc ggggtctcgg ctccagcgcg 420
   gccggctcac gcgcgatcgc cttggccctc gccgacctct tcggccacga actggccgag 480
   cacacggcgt acgaactggt gcagacggcc gagaacatgg cgcacggccg ggccagcggc 540
   gtggacgcga tgacggtcgg cgcgtcccgg ccgctgctgt tccagcaggg ccgcaccgag 600
   cgactggcca tcggctgcga cagcctgttc atcgtcgccg acagcggcgt cccgggcagc 660
   accaaggaag cggtcgagat gctgcgggag ggattcaccc gcagcgccgg aacacaggag 720
   cggttcgtcg gccgggcgac ggaactgacc gaggccgccc ggcaggccct cgccgacggc 780
   cggcccgagg agctgggctc gcagctgacg tactaccacg agctgctcca tgaggcccgc 840
   ctgagcaccg acggcatcga tgcgctggtc gaggccgcgc tgaaggcagg cagcctcgga 900
   gccaagatca ccggcggtgg tctgggcggc tgcatgatcg cacaggcccg gcccgaacag 960
   gcccgggagg tcacccggca gctccacgag gccggtgccg tacagacctg ggtcgtaccg 1020
   ctgaaagggc tcgacaacca tgcgcagtga 1050
<210> 10
   <211> 1065
   <212> DNA
   <213> Streptomyces
<400> 10
   ggaggttgtt ttatgcgcag tgaacacccg accacgaccg tgctccagtc gcgggagcag 60
   ggcagcgcgg ccggcgccac cgcggtcgcg cacccaaaca tcgcgctgat caagtactgg 120
   ggcaagcgcg acgagcggct gatcctgccc tgcaccacca gcctgtcgat gacgctggac 180
   gtcttcccca cgaccaccga ggtccggctc gaccccgccg ccgagcacga cacggccgcc 240
   ctcaacggcg aggtggccac gggcgagacg ctgcgccgca tcagcgcctt cctctccctg 300
   gtgcgggagg tggcgggcag cgaccagcgg gccgtggtgg acacccgcaa caccgtgccc 360
   accggggcgg gcctggcgtc ctccgccagc gggttcgccg ccctcgccgt cgcggccgcg 420
   gccgcctacg ggctcgaact cgacgaccgc gggctgtccc ggctggcccg acgtggatcc 480
   ggctccgcct cgcggtcgat cttcggcggc ttcgccgtct ggcacgccgg ccccgacggc 540
   acggccacgg aagcggacct cggctcctac gccgagccgg tgcccgcggc cgacctcgac 600
   ccggcgctgg tcatcgccgt ggtcaacgcc ggccccaagc ccgtctccag ccgcgaggcc 660
   atgcgccgca ccgtcgacac ctcgccgctg taccggccgt gggccgactc cagtaaggac 720
   gacctggacg agatgcgctc ggcgctgctg cgcggcgacc tcgaggccgt gggcgagatc 780
   gcggagcgca acgcgctcgg catgcacgcc accatgctgg ccgcccgccc cgcggtgcgg 840
   tacctgtcgc cggccacggt caccgtgctc gacagcgtgc tccagctccg caaggacggt 900
   gtcctggcct acgcgaccat ggacgccggt cccaacgtga aggtgctgtg ccggcgggcg 960
   gacgccgagc gggtggccga cgtcgtacgc gccgccgcgt ccggcggtca ggtcctcgtc 1020
   gccgggccgg gagacggtgc ccgcctgctg agcgagggcg catga 1065
<210> 11
   <211> 1137
   <212> DNA
   <213> Streptomyces
<400> 11
   ggaggttgtt ttatgacgac aggtcagcgc acgatcgtcc ggcacgcgcc gggcaagctg 60
   ttcgtcgcgg gcgagtacgc ggtcgtggat ccgggcaacc cggcgatcct ggtagcggtc 120
   gaccggcaca tcagcgtcac cgtgtccgac gccgacgcgg acaccggggc cgccgacgtc 180
   gtgatctcct ccgacctcgg tccgcaggcg gtcggctggc gctggcacga cggccggctc 240
   gtcgtccgcg acccggacga cgggcagcag gcgcgcagcg ccctggccca cgtggtgtcg 300
   gcgatcgaga ccgtgggccg gctgctgggc gaacgcggac agaaggtccc cgctctcacc 360
   ctctccgtca gcagccgcct gcacgaggac ggccggaagt tcggcctggg ctccagcggc 420
   gcggtgaccg tggcgaccgt agccgccgtc gccgcgttct gcggactcga actgtccacc 480
   gacgaacggt tccggctggc catgctcgcc accgcggaac tcgaccccaa gggctccggc 540
   ggggacctcg ccgccagcac ctggggcggc tggatcgcct accaggcgcc cgaccgggcc 600
   tttgtgctcg acctggcccg gcgcgtggga gtcgaccgga cactgaaggc gccctggccg 660
   gggcactcgg tgcgccgact gccggcgccc aagggcctca ccctggaggt cggctggacc 720
   ggagagcccg cctccaccgc gtccctggtg tccgatctgc accgccgcac ctggcggggc 780
   agcgcctccc accagaggtt cgtcgagacc acgaccgact gtgtccgctc cgcggtcacc 840
   gccctggagt ccggcgacga cacgagcctg ctgcacgaga tccgccgggc ccgccaggag 900
   ctggcccgcc tggacgacga ggtcggcctc ggcatcttca cacccaagct gacggcgctg 960
   tgcgacgccg ccgaagccgt cggcggcgcg gccaagccct ccggggcagg cggcggcgac 1020
   tgcggcatcg ccctgctgga cgccgaggcg tcgcgggaca tcacacatgt acggcaacgg 1080
   tgggagacag ccggggtgct gcccctgccc ctgactcctg ccctggaagg gatctaa 1137
<210> 12
   <211> 1104
   <212> DNA
   <213> Streptomyces
<400> 12
   ggaggttgtt ttatgaccag cgcccaacgc aaggacgacc acgtacggct cgccatcgag 60
   cagcacaacg cccacagcgg acgcaaccag ttcgacgacg tgtcgttcgt ccaccacgcc 120
   ctggccggca tcgaccggcc ggacgtgtcc ctggccacgt ccttcgccgg gatctcctgg 180
   caggtgccga tctacatcaa cgcgatgacc ggcggcagcg agaagaccgg cctcatcaac 240
   cgggacctgg ccaccgccgc ccgcgagacc ggcgtcccca tcgcgtccgg gtccatgaac 300
   gcgtacatca aggacccctc ctgcgccgac acgttccgtg tgctgcgcga cgagaacccc 360
   aacgggttcg tcatcgcgaa catcaacgcc accacgacgg tcgacaacgc gcagcgcgcg 420
   atcgacctga tcgaggcgaa cgccctgcag atccacatca acacggcgca ggagacgccg 480
   atgccggagg gcgaccggtc gttcgcgtcc tgggtcccgc agatcgagaa gatcgcggcg 540
   gccgtcgaca tccccgtgat cgtcaaggag gtcggcaacg gcctgagccg gcagaccatc 600
   ctgctgctcg ccgacctcgg cgtgcaggcg gcggacgtca gcggccgcgg cggcacggac 660
   ttcgcccgca tcgagaacgg ccgccgggag ctcggcgact acgcgttcct gcacggctgg 720
   gggcagtcca ccgccgcctg cctgctggac gcccaggaca tctccctgcc cgtcctcgcc 780
   tccggcggtg tgcgtcaccc gctcgacgtg gtccgcgccc tcgcgctcgg cgcccgcgcc 840
   gtcggctcct ccgccggctt cctgcgcacc ctgatggacg acggcgtcga cgcgctgatc 900
   acgaagctca cgacctggct ggaccagctg gcggcgctgc agaccatgct cggcgcgcgc 960
   accccggccg acctcacccg ctgcgacgtg ctgctccacg gcgagctgcg tgacttctgc 1020
   gccgaccggg gcatcgacac gcgccgcctc gcccagcgct ccagctccat cgaggccctc 1080
   cagacgacgg gaagcacacg atga 1104
<210> 13
   <211> 6798
   <212> DNA
   <213> Streptomyces
<400> 13
<210> 14
   <211> 26
   <212> DNA
   <213> Artificial Sequence
<220>
   <223> Description of Artificial Sequence: an artificially synthesized primer sequence
<400> 14
   ggggatcctt ggagcaaacg cactgc 26
<210> 15
   <211> 27
   <212> DNA
   <213> Artificial Sequence
<220>
   <223> Description of Artificial Sequence: an artificially synthesized primer sequence
<400> 15
   ggggatccaa aaaaggagat ttgccgc 27

### SEQUENCE LISTING

<110> CENTER FOR ADVANCED SCIENCE AND TECHNOLOGY INCUB.,
<120> A METHOD FOR SCREENING SUBSTANCES WHICH SPECIFICALLY INHIBIT THE NON-MEVALONATE PATHWAY
<130> P20557EP
<140> 01 908 140.5
   <141> 2001-02-28
<150> JP 56753/2000
   <151> 2000-03-02
<160> 15
<170> PatentIn Ver. 2.1
<210> 1
   <211> 40
   <212> DNA
   <213> Artificial Sequence
<220>
   <223> Description of Artificial Sequence: an artificially synthesized primer sequence
<400> 1
   ggaagcttgg aggttgtttt atgcagaaaa gacaaaggga 40
<210> 2
   <211> 28
   <212> DNA
   <213> Artificial Sequence
<220>
   <223> Description of Artificial Sequence: an artificially synthesized primer sequence
<400> 2
   ggtctagatc actgcgcatg gttgtcga 28
<210> 3
   <211> 40
   <212> DNA
   <213> Artificial Sequence
<220>
   <223> Description of Artificial Sequence: an artificially synthesized primer sequence
<400> 3
   ggtctagagg aggttgtttt atgcgcagtg aacacccgac 40
<210> 4
   <211> 28
   <212> DNA
   <213> Artificial Sequence
<220>
   <223> Description of Artificial Sequence: an artificially synthesized primer sequence
<400> 4
   ggagatcttc atgcgccctc gctcagca 28
<210> 5
   <211> 40
   <212> DNA
   <213> Artificial Sequence
<220>
   <223> Description of Artificial Sequence: an artificially synthesized primer sequence
<400> 5
   ggagatctgg aggttgtttt atgacgacag gtcagcgcac 40
<210> 6
   <211> 28
   <212> DNA
   <213> Artificial Sequence
<220>
   <223> Description of Artificial Sequence: an artificially synthesized primer sequence
<400> 6
   ggatcgattt agatcccttc cagggcag 28
<210> 7
   <211> 40
   <212> DNA
   <213> Artificial Sequence
<220>
   <223> Description of Artificial Sequence: an artificially synthesized primer sequence
<400> 7
   ggatcgatgg aggttgtttt atgaccagcg cccaacgcaa 40
<210> 8
   <211> 28
   <212> DNA
   <213> Artificial Sequence
<220>
   <223> Description of Artificial Sequence: an artificially synthesized primer sequence
<400> 8
   gggcatgctc atcgtgtgct tcccgtcg 28
<210> 9
   <211> 1050
   <212> DNA
   <213> Streptomyces
<400> 9
   ggaggttgtt ttatgcagaa aagacaaagg gagctgagtg cgttgacact accgacctcg 60
   gctgaggggg tatcagaaag ccaccgggcc cgctcggtcg gcatcggtcg cgcccacgcc 120
   aaggccatcc tgctgggaga gcatgcggtc gtctacggag cgccggcact cgctctgccg 180
   attccgcagc tcacggtcac ggccagcgtc ggctggtcgt ccgaggcctc cgacagtgcg 240
   ggtggcctgt cctacacgat gaccggtacg ccgtcgcggg cactggtgac gcaggcctcc 300
   gacggcctgc accggctcac cgcggaattc atggcgcgga tgggcgtgac gaacgcgccg 360
   cacctcgacg tgatcctgga cggcgcgatc ccgcacggcc ggggtctcgg ctccagcgcg 420
   gccggctcac gcgcgatcgc cttggccctc gccgacctct tcggccacga actggccgag 480
   cacacggcgt acgaactggt gcagacggcc gagaacatgg cgcacggccg ggccagcggc 540
   gtggacgcga tgacggtcgg cgcgtcccgg ccgctgctgt tccagcaggg ccgcaccgag 600
   cgactggcca tcggctgcga cagcctgttc atcgtcgccg acagcggcgt cccgggcagc 660
   accaaggaag cggtcgagat gctgcgggag ggattcaccc gcagcgccgg aacacaggag 720
   cggttcgtcg gccgggcgac ggaactgacc gaggccgccc ggcaggccct cgccgacggc 780
   cggcccgagg agctgggctc gcagctgacg tactaccacg agctgctcca tgaggcccgc 840
   ctgagcaccg acggcatcga tgcgctggtc gaggccgcgc tgaaggcagg cagcctcgga 900
   gccaagatca ccggcggtgg tctgggcggc tgcatgatcg cacaggcccg gcccgaacag 960
   gcccgggagg tcacccggca gctccacgag gccggtgccg tacagacctg ggtcgtaccg 1020
   ctgaaagggc tcgacaacca tgcgcagtga 1050
<210> 10
   <211> 1065
   <212> DNA
   <213> Streptomyces
<400> 10
   ggaggttgtt ttatgcgcag tgaacacccg accacgaccg tgctccagtc gcgggagcag 60
   ggcagcgcgg ccggcgccac cgcggtcgcg cacccaaaca tcgcgctgat caagtactgg 120
   ggcaagcgcg acgagcggct gatcctgccc tgcaccacca gcctgtcgat gacgctggac 180
   gtcttcccca cgaccaccga ggtccggctc gaccccgccg ccgagcacga cacggccgcc 240
   ctcaacggcg aggtggccac gggcgagacg ctgcgccgca tcagcgcctt cctctccctg 300
   gtgcgggagg tggcgggcag cgaccagcgg gccgtggtgg acacccgcaa caccgtgccc 360
   accggggcgg gcctggcgtc ctccgccagc gggttcgccg ccctcgccgt cgcggccgcg 420
   gccgcctacg ggctcgaact cgacgaccgc gggctgtccc ggctggcccg acgtggatcc 480
   ggctccgcct cgcggtcgat cttcggcggc ttcgccgtct ggcacgccgg ccccgacggc 540
   acggccacgg aagcggacct cggctcctac gccgagccgg tgcccgcggc cgacctcgac 600
   ccggcgctgg tcatcgccgt ggtcaacgcc ggccccaagc ccgtctccag ccgcgaggcc 660
   atgcgccgca ccgtcgacac ctcgccgctg taccggccgt gggccgactc cagtaaggac 720
   gacctggacg agatgcgctc ggcgctgctg cgcggcgacc tcgaggccgt gggcgagatc 780
   gcggagcgca acgcgctcgg catgcacgcc accatgctgg ccgcccgccc cgcggtgcgg 840
   tacctgtcgc cggccacggt caccgtgctc gacagcgtgc tccagctccg caaggacggt 900
   gtcctggcct acgcgaccat ggacgccggt cccaacgtga aggtgctgtg ccggcgggcg 960
   gacgccgagc gggtggccga cgtcgtacgc gccgccgcgt ccggcggtca ggtcctcgtc 1020
   gccgggccgg gagacggtgc ccgcctgctg agcgagggcg catga                 1065
<210> 11
   <211> 1137
   <212> DNA
   <213> Streptomyces
<400> 11
   ggaggttgtt ttatgacgac aggtcagcgc acgatcgtcc ggcacgcgcc gggcaagctg 60
   ttcgtcgcgg gcgagtacgc ggtcgtggat ccgggcaacc cggcgatcct ggtagcggtc 120
   gaccggcaca tcagcgtcac cgtgtccgac gccgacgcgg acaccggggc cgccgacgtc 180
   gtgatctcct ccgacctcgg tccgcaggcg gtcggctggc gctggcacga cggccggctc 240
   gtcgtccgcg acccggacga cgggcagcag gcgcgcagcg ccctggccca cgtggtgtcg 300
   gcgatcgaga ccgtgggccg gctgctgggc gaacgcggac agaaggtccc cgctctcacc 360
   ctctccgtca gcagccgcct gcacgaggac ggccggaagt tcggcctggg ctccagcggc 420
   gcggtgaccg tggcgaccgt agccgccgtc gccgcgttct gcggactcga actgtccacc 480
   gacgaacggt tccggctggc catgctcgcc accgcggaac tcgaccccaa gggctccggc 540
   ggggacctcg ccgccagcac ctggggcggc tggatcgcct accaggcgcc cgaccgggcc 600
   tttgtgctcg acctggcccg gcgcgtggga gtcgaccgga cactgaaggc gccctggccg 660
   gggcactcgg tgcgccgact gccggcgccc aagggcctca ccctggaggt cggctggacc 720
   ggagagcccg cctccaccgc gtccctggtg tccgatctgc accgccgcac ctggcggggc 780
   agcgcctccc accagaggtt cgtcgagacc acgaccgact gtgtccgctc cgcggtcacc 840
   gccctggagt ccggcgacga cacgagcctg ctgcacgaga tccgccgggc ccgccaggag 900
   ctggcccgcc tggacgacga ggtcggcctc ggcatcttca cacccaagct gacggcgctg 960
   tgcgacgccg ccgaagccgt cggcggcgcg gccaagccct ccggggcagg cggcggcgac 1020
   tgcggcatcg ccctgctgga cgccgaggcg tcgcgggaca tcacacatgt acggcaacgg 1080
   tgggagacag ccggggtgct gcccctgccc ctgactcctg ccctggaagg gatctaa 1137
<210> 12
   <211> 1104
   <212> DNA
   <213> Streptomyces
<400> 12
   ggaggttgtt ttatgaccag cgcccaacgc aaggacgacc acgtacggct cgccatcgag 60
   cagcacaacg cccacagcgg acgcaaccag ttcgacgacg tgtcgttcgt ccaccacgcc 120
   ctggccggca tcgaccggcc ggacgtgtcc ctggccacgt ccttcgccgg gatctcctgg 180
   caggtgccga tctacatcaa cgcgatgacc ggcggcagcg agaagaccgg cctcatcaac 240
   cgggacctgg ccaccgccgc ccgcgagacc ggcgtcccca tcgcgtccgg gtccatgaac 300
   gcgtacatca aggacccctc ctgcgccgac acgttccgtg tgctgcgcga cgagaacccc 360
   aacgggttcg tcatcgcgaa catcaacgcc accacgacgg tcgacaacgc gcagcgcgcg 420
   atcgacctga tcgaggcgaa cgccctgcag atccacatca acacggcgca ggagacgccg 480
   atgccggagg gcgaccggtc gttcgcgtcc tgggtcccgc agatcgagaa gatcgcggcg 540
   gccgtcgaca tccccgtgat cgtcaaggag gtcggcaacg gcctgagccg gcagaccatc 600
   ctgctgctcg ccgacctcgg cgtgcaggcg gcggacgtca gcggccgcgg cggcacggac 660
   ttcgcccgca tcgagaacgg ccgccgggag ctcggcgact acgcgttcct gcacggctgg 720
   gggcagtcca ccgccgcctg cctgctggac gcccaggaca tctccctgcc cgtcctcgcc 780
   tccggcggtg tgcgtcaccc gctcgacgtg gtccgcgccc tcgcgctcgg cgcccgcgcc 840
   gtcggctcct ccgccggctt cctgcgcacc ctgatggacg acggcgtcga cgcgctgatc 900
   acgaagctca cgacctggct ggaccagctg gcggcgctgc agaccatgct cggcgcgcgc 960
   accccggccg acctcacccg ctgcgacgtg ctgctccacg gcgagctgcg tgacttctgc 1020
   gccgaccggg gcatcgacac gcgccgcctc gcccagcgct ccagctccat cgaggccctc 1080
   cagacgacgg gaagcacacg atga 1104
<210> 13
   <211> 6798
   <212> DNA
   <213> Streptomyces
<400> 13
   tacgtacttc cctggcgtgt gcagcggttg acgcgccgtg ccctcgctgc gagcggcgcg 60
   cacatctgac gtcctgcttt attgctttct cagaactcgg gacgaagcga tcccatgatc 120
   acgcgatctc catgcagaaa agacaaaggg agctgagtgc gttgacacta ccgacctcgg 180
   ctgagggggt atcagaaagc caccgggccc gctcggtcgg catcggtcgc gcccacgcca 240
   aggccatcct gctgggagag catgcggtcg tctacggagc gccggcactc gctctgccga 300
   ttccgcagct cacggtcacg gccagcgtcg gctggtcgtc cgaggcctcc gacagtgcgg 360
   gtggcctgtc ctacacgatg accggtacgc cgtcgcgggc actggtgacg caggcctccg 420
   acggcctgca ccggctcacc gcggaattca tggcgcggat gggcgtgacg aacgcgccgc 480
   acctcgacgt gatcctggac ggcgcgatcc cgcacggccg gggtctcggc tccagcgcgg 540
   ccggctcacg cgcgatcgcc ttggccctcg ccgacctctt cggccacgaa ctggccgagc 600
   acacggcgta cgaactggtg cagacggccg agaacatggc gcacggccgg gccagcggcg 660
   tggacgcgat gacggtcggc gcgtcccggc cgctgctgtt ccagcagggc cgcaccgagc 720
   gactggccat cggctgcgac agcctgttca tcgtcgccga cagcggcgtc ccgggcagca 780
   ccaaggaagc ggtcgagatg ctgcgggagg gattcacccg cagcgccgga acacaggagc 840
   ggttcgtcgg ccgggcgacg gaactgaccg aggccgcccg gcaggccctc gccgacggcc 900
   ggcccgagga gctgggctcg cagctgacgt actaccacga gctgctccat gaggcccgcc 960
   tgagcaccga cggcatcgat gcgctggtcg aggccgcgct gaaggcaggc agcctcggag 1020
   ccaagatcac cggcggtggt ctgggcggct gcatgatcgc acaggcccgg cccgaacagg 1080
   cccgggaggt cacccggcag ctccacgagg ccggtgccgt acagacctgg gtcgtaccgc 1140
   tgaaagggct cgacaaccat gcgcagtgaa cacccgacca cgaccgtgct ccagtcgcgg 1200
   gagcagggca gcgcggccgg cgccaccgcg gtcgcgcacc caaacatcgc gctgatcaag 1260
   tactggggca agcgcgacga gcggctgatc ctgccctgca ccaccagcct gtcgatgacg 1320
   ctggacgtct tccccacgac caccgaggtc cggctcgacc ccgccgccga gcacgacacg 1380
   gccgccctca acggcgaggt ggccacgggc gagacgctgc gccgcatcag cgccttcctc 1440
   tccctggtgc gggaggtggc gggcagcgac cagcgggccg tggtggacac ccgcaacacc 1500
   gtgcccaccg gggcgggcct ggcgtcctcc gccagcgggt tcgccgccct cgccgtcgcg 1560
   gccgcggccg cctacgggct cgaactcgac gaccgcgggc tgtcccggct ggcccgacgt 1620
   ggatccggct ccgcctcgcg gtcgatcttc ggcggcttcg ccgtctggca cgccggcccc 1680
   gacggcacgg ccacggaagc ggacctcggc tcctacgccg agccggtgcc cgcggccgac 1740
   ctcgacccgg cgctggtcat cgccgtggtc aacgccggcc ccaagcccgt ctccagccgc 1800
   gaggccatgc gccgcaccgt cgacacctcg ccgctgtacc ggccgtgggc cgactccagt 1860
   aaggacgacc tggacgagat gcgctcggcg ctgctgcgcg gcgacctcga ggccgtgggc 1920
   gagatcgcgg agcgcaacgc gctcggcatg cacgccacca tgctggccgc ccgccccgcg 1980
   gtgcggtacc tgtcgccggc cacggtcacc gtgctcgaca gcgtgctcca gctccgcaag 2040
   gacggtgtcc tggcctacgc gaccatggac gccggtccca acgtgaaggt gctgtgccgg 2100
   cgggcggacg ccgagcgggt ggccgacgtc gtacgcgccg ccgcgtccgg cggtcaggtc 2160
   ctcgtcgccg ggccgggaga cggtgcccgc ctgctgagcg agggcgcatg acgacaggtc 2220
   agcgcacgat cgtccggcac gcgccgggca agctgttcgt cgcgggcgag tacgcggtcg 2280
   tggatccggg caacccggcg atcctggtag cggtcgaccg gcacatcagc gtcaccgtgt 2340
   ccgacgccga cgcggacacc ggggccgccg acgtcgtgat ctcctccgac ctcggtccgc 2400
   aggcggtcgg ctggcgctgg cacgacggcc ggctcgtcgt ccgcgacccg gacgacgggc 2460
   agcaggcgcg cagcgccctg gcccacgtgg tgtcggcgat cgagaccgtg ggccggctgc 2520
   tgggcgaacg cggacagaag gtccccgctc tcaccctctc cgtcagcagc cgcctgcacg 2580
   aggacggccg gaagttcggc ctgggctcca gcggcgcggt gaccgtggcg accgtagccg 2640
   ccgtcgccgc gttctgcgga ctcgaactgt ccaccgacga acggttccgg ctggccatgc 2700
   tcgccaccgc ggaactcgac cccaagggct ccggcgggga cctcgccgcc agcacctggg 2760
   gcggctggat cgcctaccag gcgcccgacc gggcctttgt gctcgacctg gcccggcgcg 2820
   tgggagtcga ccggacactg aaggcgccct ggccggggca ctcggtgcgc cgactgccgg 2880
   cgcccaaggg cctcaccctg gaggtcggct ggaccggaga gcccgcctcc accgcgtccc 2940
   tggtgtccga tctgcaccgc cgcacctggc ggggcagcgc ctcccaccag aggttcgtcg 3000
   agaccacgac cgactgtgtc cgctccgcgg tcaccgccct ggagtccggc gacgacacga 3060
   gcctgctgca cgagatccgc cgggcccgcc aggagctggc ccgcctggac gacgaggtcg 3120
   gcctcggcat cttcacaccc aagctgacgg cgctgtgcga cgccgccgaa gccgtcggcg 3180
   gcgcggccaa gccctccggg gcaggcggcg gcgactgcgg catcgccctg ctggacgccg 3240
   aggcgtcgcg ggacatcaca catgtacggc aacggtggga gacagccggg gtgctgcccc 3300
   tgcccctgac tcctgccctg gaagggatct aagaatgacc agcgcccaac gcaaggacga 3360
   ccacgtacgg ctcgccatcg agcagcacaa cgcccacagc ggacgcaacc agttcgacga 3420
   cgtgtcgttc gtccaccacg ccctggccgg catcgaccgg ccggacgtgt ccctggccac 3480
   gtccttcgcc gggatctcct ggcaggtgcc gatctacatc aacgcgatga ccggcggcag 3540
   cgagaagacc ggcctcatca accgggacct ggccaccgcc gcccgcgaga ccggcgtccc 3600
   catcgcgtcc gggtccatga acgcgtacat caaggacccc tcctgcgccg acacgttccg 3660
   tgtgctgcgc gacgagaacc ccaacgggtt cgtcatcgcg aacatcaacg ccaccacgac 3720
   ggtcgacaac gcgcagcgcg cgatcgacct gatcgaggcg aacgccctgc agatccacat 3780
   caacacggcg caggagacgc cgatgccgga gggcgaccgg tcgttcgcgt cctgggtccc 3840
   gcagatcgag aagatcgcgg cggccgtcga catccccgtg atcgtcaagg aggtcggcaa 3900
   cggcctgagc cggcagacca tcctgctgct cgccgacctc ggcgtgcagg cggcggacgt 3960
   cagcggccgc ggcggcacgg acttcgcccg catcgagaac ggccgccggg agctcggcga 4020
   ctacgcgttc ctgcacggct gggggcagtc caccgccgcc tgcctgctgg acgcccagga 4080
   catctccctg cccgtcctcg cctccggcgg tgtgcgtcac ccgctcgacg tggtccgcgc 4140
   cctcgcgctc ggcgcccgcg ccgtcggctc ctccgccggc ttcctgcgca ccctgatgga 4200
   cgacggcgtc gacgcgctga tcacgaagct cacgacctgg ctggaccagc tggcggcgct 4260
   gcagaccatg ctcggcgcgc gcaccccggc cgacctcacc cgctgcgacg tgctgctcca 4320
   cggcgagctg cgtgacttct gcgccgaccg gggcatcgac acgcgccgcc tcgcccagcg 4380
   ctccagctcc atcgaggccc tccagacgac gggaagcaca cgatgacgga aacgcacgcc 4440
   atagccgggg tcccgatgag gtgggtggga ccccttcgta tttccgggaa cgtcgccgag 4500
   accgagaccc aggtcccgct cgccacgtac gagtcgccgc tgtggccgtc ggtgggccgc 4560
   ggggcgaagg tctcccggct gacggagaag ggcatcgtcg ccaccctcgt cgacgagcgg 4620
   atgacccgct cggtgatcgt cgaggcgacg gacgcgcaga ccgcgtacat ggccgcgcag 4680
   accatccacg cccgcatcga cgagctgcgc gaggtggtgc gcggctgcag ccggttcgcc 4740
   cagctgatca acatcaagca cgagatcaac gcgaacctgc tgttcatccg gttcgagttc 4800
   accaccggtg acgcctccgg ccacaacatg gccacgctcg cctccgatgt gctcctgggg 4860
   cacctgctgg agacgatccc tggcatctcc tacggctcga tctccggcaa ctactgcacg 4920
   gacaagaagg ccaccgcgat caacggcatc ctcggccgcg gcaagaacgt gatcaccgag 4980
   ctgctggtgc cgcgggacgt cgtcgagaac aacctgcaca ccacggctgc caagatcgtc 5040
   gagctgaaca tccgcaagaa cctgctcggc accctgctcg ccggcggcat ccgctcggcc 5100
   aacgcccact tcgcgaacat gctgctcggc ttctacctgg ccaccggcca ggacgccgcc 5160
   aacatcgtcg agggctcgca gggcgtcgtc atggccgagg accgcgacgg cgacctctac 5220
   ttcgcctgca ccctgccgaa cctgatcgtc ggcacggtcg gcaacggcaa gggtctcggc 5280
   ttcgtggaga cgaacctcgc ccggctcggc tgccgagccg accgcgaacc cggggagaac 5340
   gcccgccgcc tcgccgtcat cgcggcagcg accgtgctgt gcggtgaact ctcgctgctc 5400
   gcggcacaga cgaacccggg cgaactcatg cgcgcgcacg tccagctgga acgcgacaac 5460
   aagaccgcaa aggttggtgc atagggcatg tccatctcca taggcattca cgacctgtcg 5520
   ttcgccacaa ccgagttcgt cctgccgcac acggcgctcg ccgagtacaa cggcaccgag 5580
   atcggcaagt accacgtcgg catcggccag cagtcgatga gcgtgccggc cgccgacgag 5640
   gacatcgtga ccatggccgc gaccgcggcg cggcccatca tcgagcgcaa cggcaagagc 5700
   cggatccgca cggtcgtgtt cgccacggag tcgtcgatcg accaggcgaa ggcgggcggc 5760
   gtgtacgtgc actccctgct ggggctggag tcggcctgcc gggtcgtcga gctgaagcag 5820
   gcctgctacg gggccaccgc cgcccttcag ttcgccatcg gcctggtgcg gcgcgacccc 5880
   gcccagcagg tcctggtcat cgccagtgac gtctccaagt acgagctgga cagccccggc 5940
   gaggcgaccc agggcgcggc cgcggtggcc atgctggtcg gcgccgaccc ggccctgctg 6000
   cgtatcgagg agccgtcggg cctgttcacc gccgacgtca tggacttctg gcggcccaac 6060
   tacctcacca ccgctctggt cgacggccag gagtccatca acgcctacct gcaggccgtc 6120
   gagggcgcct ggaaggacta cgcggagcag gacggccggt cgctggagga gttcgcggcg 6180
   ttcgtctacc accagccgtt cacgaagatg gcctacaagg cgcaccgcca cctgctgaac 6240
   ttcaacggct acgacaccga caaggacgcc atcgagggcg ccctcggcca gacgacggcg 6300
   tacaacaacg tcatcggcaa cagctacacc gcgtcggtgt acctgggcct ggccgccctg 6360
   ctcgaccagg cggacgacct gacgggccgt tccatcggct tcctgagcta cggctcgggc 6420
   agcgtcgccg agttcttctc gggcaccgtc gtcgccgggt accgcgagcg tctgcgcacc 6480
   gaggcgaacc aggaggcgat cgcccggcgc aagagcgtcg actacgccac ctaccgcgag 6540
   ctgcacgagt acacgctccc gtccgacggc ggcgaccacg ccaccccggt gcagaccacc 6600
   ggccccttcc ggctggccgg gatcaacgac cacaagcgca tctacgaggc gcgctagcga 6660
   cacccctcgg caacggggtg cgccactgtt cggcgcaccc cgtgccgggc tttcgcacag 6720
   ctattcacga ccatttgagg ggcgggcagc cgcatgaccg acgtccgatt ccgcattatc 6780
   ggtacgggtg cctacgta                                               6798
<210> 14
   <211> 26
   <212> DNA
   <213> Artificial Sequence
<220>
   <223> Description of Artificial Sequence: an artificially synthesized primer sequence
<400> 14
   ggggatcctt ggagcaaacg cactgc 26
<210> 15
   <211> 27
   <212> DNA
   <213> Artificial Sequence
<220>
   <223> Description of Artificial Sequence: an artificially synthesized primer sequence
<400> 15
   ggggatccaa aaaaggagat ttgccgc 27

## Description

### Technical Field

The present invention relates to a method for screening substances which specifically inhibit the non-mevalonate pathway. More specifically, the present invention relates to a method for screening substances which specifically inhibit the non-mevalonate pathway by using an organism capable of using both the mevalonate pathway and the non-mevalonate pathway, a transformant used for the above screening method, and the substances useful as anti-bacterial agent, herbicide or anti-malarial agent selected by the above screening method.

### Background Art

In many eubacteria including *Bacillus subtilis* or the plastid of higher plants, only the non-mevalonate pathway functions as a biosynthetic pathway of isopentenyl diphosphate (IPP) and dimethylallyl diphosphate (DMAPP) which are essential compounds for growth (Comprehensive Natural Products Chemistry. Elsevier, Amsterdam, Vol. 2, 45, 1999). When the non-mevalonate pathway of the above organisms is blocked, these organisms cannot carry out the biosynthesis of isopentenyl diphosphate (IPP) or dimethylallyl diphosphate (DMAPP), and accordingly the organisms become unable to grow. Therefore, a compound having an activity of inhibiting any enzyme on the non-mevalonate pathway is useful as anti-bacterial agent, herbicide or anti-malarial agent.

Regarding the non-mevalonate pathway, the first stage wherein 1-deoxy-D-xylulose-5-phosphate (DXP) is generated by condensation of pyruvic acid and glyceraldehyde-3-phosphate (Proc. Natl. Acad. Sci. USA, 94, 12857, (1997)), the second stage wherein 2-C-methyl-D-erythritol 4-phosphate (MEP) is generated from DXP (Tetrahedron Lett., 39, 4509, (1998)), the third stage wherein 4-(cytidine 5'-diphospho)-2-C-methyl-D-erythritol (CDP-ME) is generated from MEP (Tetrahedron Lett., 41, 703, (2000)), and the fourth stage wherein 2-phospho-4-(cytidine 5'-diphospho)-2-C-methyl-D-erythritol (CDP-ME2P) is generated from CDP-ME (Tetrahedron Lett., under contribution) have been clarified, and enzymes catalyzing each of the above reactions and genes encoding the enzymes have been identified. However, there are no findings regarding a reaction pathway from the fourth stage to IPP, enzymes catalyzing such reactions, and genes encoding those enzymes.

Presently, there is a report regarding a method for screening a compound which has an activity of specifically inhibiting DXP reductoisomerase that is an enzyme at the second stage on the non-mevalonate pathway (Proc. Natl. Acad. Sci. USA 95, 9879, (1998)). However, there are no reports regarding a method for screening compounds having an activity which inhibits an enzyme at other stages on the non-mevalonate pathway. A method of purifying each enzyme on the non-mevalonate pathway and screening compounds having an activity of inhibiting each enzyme may be considered, but the operation of purifying each enzyme and analyzing an activity of inhibiting the enzyme is complicated, and such an operation cannot be an efficient screening method. Moreover, since an enzyme catalyzing enzyme reactions after 2-phospho-4-(cytidine 5'-diphospho)-2-C-methyl-D-erythritol (CDP-ME2P) has not been identified at the moment, a substance which inhibits these enzyme reactions cannot be screened by the above described method.

As another biosynthetic pathway of IPP which is different from the non-mevalonate pathway, there is known the mevalonate pathway. Specifically, in eukaryote such as animals or yeast, IPP is biosynthesized from acetyl CoA via mevalonic acid. As stated above, many eubacteria including *Bacillus subtilis* or the plastid of higher plants do not use the mevalonate pathway, but use the non-mevalonate pathway.

It is known that Streptomyces, *Streptomyces* sp. CL190 (J. Antibiot. 43:444, 1990) synthesizes IPP via mevalonic acid (Tetrahedron Lett. 31:6025, 1990 and Tetrahedron Lett. 37:7979, 1996). Up to now, the present inventors have cloned a gene (hmgr) which encodes 3-hydroxy-3-methylglutaryl-CoA(HMG-CoA) reductase, which is an enzyme catalyzing one reaction on the mevalonate pathway, from *Streptomyces* sp. CL190 (J. Bacteriol. 181:1256, 1999).

The document Kuzuyama et al. ("Fosmidomycin, a Specific Inhibitor of 1-Deoxy-D-Xylulose 5-Phosphate Reductoisomerase in the Nonmevalonate Pathway for Terpenoid Biosynthesis", Tetrahedron Letters, vol. 39 (43), 1998, pages 7913-7916) describes the substance fosmidomycin which is specifically inhibiting the non-mevalonate pathway.

The document Hassan et al. ("Inhibitors of the Nonmevalonate Pathway of Isoprenoid Biosynthesis as Antimalarial Drugs", Science, vol. 285, 1999, pages 1573-1576) describes the inhibition of the recombinant *P. falciparum* DOXP reductoisomerase which is an enzyme of the non-mevalonate pathway by the substance fosmidomycin.

The international publication WO 00/01650 A1 relates to a method of vitamin production. More specifically WO 00/01650 A1 describes transformants obtained by introducing enzymes for the mevalonate pathway into a host organism (namely funghi and yeast) already having the mevalonate pathway in the wild type. In contrast, the present invention provides a transformant which is obtained by introducing DNA encoding all enzymes for using the mevalonate pathway as a biosynthetic pathway of isopentenyl diphosphate and dimethylallyl diphosphate in the presence of mevalonic acid, into a host having the non-mevalonate pathway, only.

The document Dairi et al. ("Cloning of the gene encoding 3-hydroxy-3-methylglutaryl coenzyme A reductase from terpenoid antibiotic-producing *Streptomyces* strains", Mol. Gen. Genet., vol. 262, January 2000, p.957-964) discloses a microorganism *(Streptomyces griseolosporeus* MF730-N6) which uses both the mevalonate pathway and the non-mevalonate pathway. In contrast to the present invention the document Dairy et al. provides a transformant wherein the strain *Streptomyces griseolosporeus* MF730-N6 originally uses both pathways. Further, the transformant disclosed in Dairi et al. was not constructed by introducing DNA encoding all enzymes for the mevalonate pathway, but only using the gene encoding HMG-CoA reductase.

### Disclosure of the Invention

The first object to be achieved by the present invention is to provide a novel method for screening anti-bacterial agent, herbicide or anti-malarial agents which can specifically inhibit any enzyme reaction on the non-mevalonate pathway for synthesizing isopentenyl diphosphate and dimethylallyl diphosphate.

The second object to be achieved by the present invention is to provide an efficient method for screening anti-bacterial agent, herbicide or anti-malarial agent which can specifically inhibit any enzyme reaction on the non-mevalonate pathway for synthesizing isopentenyl diphosphate and dimethylallyl diphosphate.

The third object to be achieved by the present invention is to provide a method for screening anti-bacterial agent, herbicide or anti-malarial agents which can specifically inhibit an unknown enzyme reaction on the non-mevalonate pathway for synthesizing isopentenyl diphosphate and dimethylallyl diphosphate.

The fourth object to be achieved by the present invention is to provide a method for screening anti-bacterial agent, herbicide or anti-malarial agents which can specifically inhibit an enzyme reaction on the non-mevalonate pathway for synthesizing isopentenyl diphosphate and dimethylallyl diphosphate, by using a group of Streptomyces-derived genes which encode the mevalonate pathway.

As a result of intensive research to achieve the aforementioned objects, the present inventors have first integrated, into *Bacillus subtilis,* DNA encoding mevalonate kinase, phosphomevalonate kinase, diphosphomevalonate decarboxylase and IPP isomerase, i.e., an enzyme which catalyzes the conversion of isopentenyl diphosphate (IPP) and dimethylallyl diphosphate (DMAPP), which are a group of Streptomyces-derived genes involved in the mevalonate pathway. Then, these genes were induced with IPTG to produce a transformant of *Bacillus subtilis* which can use the mevalonate pathway in the presence of mevalonic acid. Subsequently, the growth of *Bacillus subtilis* has been analyzed by culturing the thus obtained transformant of *Bacillus subtilis* together with fosmidomycin (a test substance) in the presence of mevalonic acid and IPTG (i.e. under an environment where the mevalonate pathway can be used) or in the absence of mevalonic acid and IPTG (i.e. under an environment where the mevalonate pathway cannot be used). As a result, it was confirmed that fosmidomycin does not show an anti-bacterial activity in the presence of mevalonic acid and IPTG, but shows an anti-bacterial activity in the absence of mevalonic acid and IPTG The present invention has been completed on the basis of these findings.

Thus, according to the present invention, there is provided a method for screening substances which specifically inhibit the non-mevalonate pathway by using an organism capable of using both the mevalonate pathway and the non-mevalonate pathway as biosynthetic pathways of isopentenyl diphosphate (IPP) and dimethylallyl diphosphate (DMAPP), wherein said organism is capable of using the mevalonate pathway only in the presence of mevalonic acid, wherein the organism is a transformant obtained by introducing DNA encoding all enzymes necessary for using the mevalonate pathway in the presence of mevalonic acid, into a host having the non-mevalonate pathway, wherein said enzymes are at least phosphomevalonate kinase, diphosphomevalonate decarboxylase, mevalonate kinase and IPP isomerase, and
wherein the organism is cultured with a test substance in the presence or absence of mevalonic acid, and wherein said test substance which does not inhibit the growth of the organism in the presence of mevalonic acid but inhibits the growth of the organism in the absence of mevalonic acid is indicating that said test substance inhibits specifically the non-mevalonate pathway and said test substance is selected as a candidate substance.

Preferably, the DNA encoding the enzymes necessary for using the mevalonate pathway in the presence of mevalonic acid is any of the followings:
(1) DNA having all nucleotide sequences of SEQ ID NOS: 9 to 12;
(2) DNA which has a deletion, substitution, addition and/or insertion of one or several nucleotides in the DNA of (1) above, and can allow the mevalonate pathway to function in the presence of mevalonic acid; or
(3) DNA which can hybridize with the DNA of (1) above under stringent condition, and can allow the mevalonate pathway to function in the presence of mevalonic acid.

In another embodiment of the present invention, the is provided a method for screening substances which specifically inhibit the non-mevalonate pathway by using an organism capable of using both the mevalonate pathway and the non-mevalonate pathway as biosynthetic pathways of isopentenyl diphosphate (IPP) and dimethylallyl diphosphate (DMAPP),
wherein the organism capable of using both the mevalonate pathway and the non-mevalonate pathway is an organism capable of using the mevalonate pathway in the absence of mevalonic acid, and wherein said organism is a transformant obtained by introducing DNA encoding all enzymes necessary for using the mevalonate pathway in the absence of mevalonic acid, into a host having the non-mevalonate pathway, wherein said enzymes are at least HMG-CoA synthase, HMG-CoA reductase, phosphomevalonate kinase, diphosphomevalonate decarboxylase, mevalonate kinase and IPP isomerase, and
wherein the organism capable of using both the mevalonate pathway and the non-mevalonate pathway and an organism incapable of using the mevalonate pathway but capable of using the non-mevalonate pathway are each cultured with a test substance, and wherein said test substance which does not inhibit the growth of the organism capable of using both the mevalonic pathway and the non-mevalonic pathway but inhibits the growth of the organism incapable of using the mevalonate pathway but capable of using the non-mevalonate pathway is indicating that said test substance inhibits specifically the non-mevalonate pathway and said test substance is selected as candidate substance.

Preferably, the DNA encoding enzymes necessary for using the mevalonate pathway in the absence of mevalonic acid is any of the followings:
(1) DNA having a nucleotide sequence of SEQ ID NO: 13;
(2) DNA which has a deletion, substitution, addition and/or insertion of one or several nucleotides in the nucleotide sequence of SEQ ID NO: 13, and can allow the mevalonate pathway to function in the absence of mevalonic acid; or
(3) DNA which can hybridize with the DNA of (1) above under stringent condition, and can allow the mevalonate pathway to function in the absence of mevalonic acid.

In one embodiment of the present invention, an organism not having IPP isomerase is used as the organism.

The organism is preferably a microorganism, more preferably a bacterium, further preferably a Gram-positive bacterium, and particularly preferably *Bacillus subtilis.*

The method for screening according to the present invention is preferably used for the screening of candidate compounds for anti-bacterial agent, herbicide or anti-malarial agents.

According to another aspect of the present invention, there is provided a transformant which is obtained by introducing DNA encoding all enzymes necessary for using the mevalonate pathway as a biosynthetic pathway of isopentenyl diphosphate (IPP) and dimethylallyl diphosphate (DMAPP) in the presence of mevalonic acid, into a host having the non-mevalonate pathway, only wherein said enzymes are at least phosphomevalonate kinase, diphosphomevalonate decarboxylase, mevalonate kinase and IPP isomerase.

Preferably, the DNA encoding the enzymes necessary for using the mevalonate pathway in the presence of mevalonic acid is any of the followings:
(1) DNA having all nucleotide sequences of SEQ ID NOS: 9 to 12;
(2) DNA which has a deletion, substitution, addition and/or insertion of one or several nucleotides in the DNA of (1) above, and can allow the mevalonate pathway to function in the presence of mevalonic acid; or
(3) DNA which can hybridize with the DNA of (1) above under stringent condition, and can allow the mevalonate pathway to function in the presence of mevalonic acid.

According to the above methods of the present invention, substances can be found which specifically inhibit the non-mevalonate pathway, which are selected by the aforementioned screening method of the present invention. These substances to be found which specifically inhibit the non-mevalonate pathway are preferably substances having anti-bacterial activity, herbicide activity or anti-malarial activity.

### Best Mode for Carrying Out the Invention

The embodiments and methods for carrying out the present invention will be explained in detail below.

### (A) The embodiment of the screening method of the present invention

The method for screening substances which specifically inhibit the non-mevalonate pathway according to the present invention is characterized in that it uses an organism capable of using both the mevalonate pathway and the non-mevalonate pathway as biosynthetic pathways of isopentenyl diphosphate (IPP) and dimethylallyl diphosphate (DMAPP).

The kind of the "organism" in the present specification is not particularly limited, but it is preferably an organism with which it can be simply examined whether or not a test substance has an activity of specifically inhibiting the non-mevalonate pathway. Specifically, a microorganism or cultured cell is preferable. The kind of an microorganism is not particularly limited, but it is preferably a bacterium, more preferably a Gram-positive bacterium, and particularly preferably *Bacillus subtilis* (Basillus). Moreover, a cultured cell derived from a plant is preferable as a cultured cell.

In the screening method of the present invention, there is used an organism capable of using both the mevalonate pathway and the non-mevalonate pathway. A transformant capable of using both the mevalonate pathway and the non-mevalonate pathway can be constructed by introducing DNA encoding enzymes which allow the mevalonate pathway to function into a host with using a bacterium originally having the non-mevalonate pathway as the host.

As an organism capable of using both the mevalonate pathway and the non-mevalonate pathway as biosynthetic pathways of isopentenyl diphosphate (IPP) and dimethylallyl diphosphate (DMAPP), there may used an organism capable of using the mevalonate pathway only in the presence of mevalonic acid d, or there may be used an organism capable of using the mevalonate pathway even in the absence of mevalonic acid.

Examples of the organism capable of using the mevalonate pathway only in the presence of mevalonic acid include a transformant which is obtained by introducing DNA encoding all enzymes necessary for using the mevalonate pathway in the presence of mevalonic acid into a host having the non-mevalonate pathway. The term "enzymes necessary for using the mevalonate pathway in the presence of mevalonic acid" used herein comprises at least phosphomevalonate kinase, diphosphomevalonate decarboxylase, mevalonate kinase and IPP isomerase. Examples of DNA encoding these enzymes include:
(1) DNA having all nucleotide sequences of SEQ ID NOS: 9 to 12;
(2) DNA which has a deletion, substitution, addition and/or insertion of one or several nucleotides in the DNA of (1) above, and can allow the mevalonate pathway to function in the presence of mevalonic acid; or
(3) DNA which can hybridize with the DNA of (1) above under stringent condition, and can allow the mevalonate pathway to function in the presence of mevalonic acid.

Moreover, examples of the organism capable of using the mevalonate pathway in the absence of mevalonic acid include a transformant which is obtained by introducing DNA encoding all enzymes necessary for using the mevalonate pathway in the absence of mevalonic acid into a host having the non-mevalonate pathway. The term "enzymes necessary for using the mevalonate pathway in the absence of mevalonic acid" used herein comprises at least HMG-CoA synthase, HMG-CoA reductase, phosphomevalonate kinase, diphosphomevalonate decarboxylase, mevalonate kinase and IPP isomerase. Examples of DNA encoding these enzymes include:
(1) DNA having a nucleotide sequence of SEQ ID NO: 13;
(2) DNA which has a deletion, substitution, addition and/or insertion of one or several nucleotides in the nucleotide sequence of SEQ ID NO: 13, and can allow the mevalonate pathway to function in the absence of mevalonic acid; or
(3) DNA which can hybridize with the DNA of (1) above under stringent condition, and can allow the mevalonate pathway to function in the absence of mevalonic acid.

The above-described DNA sequence includes genes which encode enzymes involved in the mevalonate pathway existing in Streptomyces, and the cloning of the genes is described in Japanese Patent Application No. 11-348375 (filed December 8, 1999).

The term "a deletion, substitution, addition and/or insertion of one or several nucleotides" in the present specification means that any given number of nucleotides, for example, 1 to 20, preferably 1 to 15, more preferably 1 to 10, and further preferably 1 to 5 nucleotides are deleted, substituted, added and/or inserted.

The term "DNA which can hybridize with ... under stringent condition" in the present specification means DNA which is obtained by using colony hybridization method, plaque hybridization method or Southern blot hybridization method using the DNA as a probe. Specifically, such DNA can be identified by performing hybridization at 65°C in the presence of 0.7 to 1.0 M NaCl using a filter on which DNA derived from a colony or plaque or its DNA fragment is fixed, and then washing the filter with about 0.1 to 2 x SSC solution (the composition of 1 x SSC solution is 150 mM sodium chloride and 15 mM sodium citrate) under a condition of 65°C. The hybridization can be carried out according to the method described in Molecular Cloning: A Laboratory Manual, 2^{nd} Ed., Cold Spring Harbor Laboratory, Cold Spring Harbor, N.Y., 1989 (hereinafter, abbreviated as "Molecular Cloning 2^{nd} Ed"), etc.

Examples of DNA which can hybridize under stringent condition include DNA having a certain or more homology with the nucleotide sequence of DNA used as a probe, and the homology is for example 60% or more, preferably 70% or more, more preferably 80% or more, further preferably 90% or more, particularly preferably 95% or more, and most preferably 98% or more.

The "mevalonate pathway" in the present specification is a pathway in which IPP is biosynthesized by the steps of:
(1) converting acetoacetyl CoA into HMG-CoA (catalyzed by HMG-CoA synthase),
(2) converting HMG-CoA into mevalonic acid (catalyzed by HMG-CoA reductase),
(3) converting mevalonic acid into pyrophosphomevalonic acid (catalyzed and controlled by mevalonate (MVA) kinase and phosphomevalonate (PMVA) kinase), and
(4) converting pyrophosphomevalonic acid into isopentenyl pyrophosphate (IPP) (catalyzed by diphosphomevalonate (PMVA) decarboxylase),
   and a pathway in which DMAPP is further biosynthesized by the step of:
(5) converting IPP into DMAPP (catalyzed by IPP isomerase).

Accordingly, examples of enzymes necessary for allowing the mevalonate pathway to function in the presence of mevalonic acid include at least PMVA kinase, PMVA decarboxylase, MVA kinase and IPP isomerase, and enzymes necessary for allowing the mevalonate pathway to function in the absence of mevalonic acid include HMG-CoA reductase and HMG-CoA synthase in addition to the above mentioned enzymes.

### (B) Method for constructing an organism capable of using both the mevalonate pathway and the non-mevalonate pathway as biosynthetic pathways of isopentenyl diphosphate (IPP) and dimethylallyl diphosphate (DMAPP)

Up till now, the present inventors have cloned a gene (hmgr) which encodes an enzyme which catalyzes one reaction on the mevalonate pathway, 3-hydroxy-3-methylglutaryl-CoA (HMG-CoA) reductase, from an actinomycete, *Streptomyces* sp. CL190 (J. Bacteriol. 181:1256, 1999). The DNA having a nucleotide sequence of SEQ ID NO: 13 in the present specification can be obtained by using this hmgr gene as a probe. Specifically, the DNA can be obtained by the following procedure.

*Streptomyces* sp. CL 190 is cultured in an appropriate medium such as a GPY medium (1% glucose, 0.4% polypeptone, 0.4% yeast extract, 0.5% MgSO₄·7H₂O, 0.1% K₂HPO₄) at an appropriate temperature (e.g. 30°C) for several days. After culturing, cells are collected from the obtained culture solution by centrifugal separation, and chromosomal DNA is then isolated and purified from the cells according to a conventional method (Molecular Cloning 2^{nd} Ed). The thus obtained chromosomal DNA is cleaved with an appropriate restriction enzyme (e.g. SnaBI), and is then subjected to Southern hybridization using the hmgr gene as a probe (Molecular Cloning 2^{nd} Ed). As a result of Southern hybridization, the signal of a probe is detected at a specific position (for example, when SnaBI is used as a restriction enzyme for the digestion of chromosomal DNA, the signal is detected at the position of 6.7 kb).

Subsequently, the chromosomal DNA of *Streptomyces* sp. CL 190 is cleaved again with the same restriction enzyme (e.g. SnaBI) as described above, and is then subjected to agarose gel electrophoresis. As a result of Southern hybridization, the signal of a probe is detected at a specific position (when SnaBI is used as a restriction enzyme, the signal is detected at the position of 6.7 kb). A DNA fragment corresponding to the position is extracted and collected from the agarose gel. The collected DNA fragment is blunt-ended by using T4 DNA polymerase (purchased from Takara Shuzo Co., Ltd.), and then inserted into an appropriate plasmid (e.g. pUC118, etc.) to produce the chromosomal DNA library of *Streptomyces* sp. CL 190. Using this chromosomal DNA library, a suitable host (e.g. *E. coli* JM 109, etc.) is transformed according to a conventional method (Molecular Cloning 2^{nd} Ed), and the transformants are screened by a colony hybridization method using the hmgr gene as a probe, so that the transformant of *E. coli* having a plasmid containing the hmgr gene can be isolated. A plasmid is extracted from the isolated transformant according to a conventional method, so that a DNA fragment containing the hmgr gene can be isolated. An example of DNA isolated by the above-described method includes DNA having a nucleotide sequence of SEQ ID NO: 13.

Moreover, using the chromosomal DNA of *Streptomyces* sp. CL 190 as a template, PCR is carried out using sense primers and antisense primers having the combinations of the nucleotide sequences of SEQ ID NOS: 1 and 2, SEQ ID NOS: 3 and 4, SEQ ID NOS: 5 and 6, and SEQ ID NOS: 7 and 8, respectively, so as to amplify DNA fragments encoding phosphomevalonate kinase (SEQ ID NO: 9), diphosphomevalonate decarboxylase (SEQ ID NO: 10), mevalonate kinase (SEQ ID NO: 11) and Protein X (IPP isomerase) (SEQ ID NO: 12).

PCR can be carried out, for example, using TaKaRa LA-PCR^{™} Kit Ver. 2 (Takara Shuzo Co., Ltd.), Expand^{™} High-Fidelity PCR System (Boehringer Mannheim), or the like, and using DNA Thermal Cycler (Perkin Elmer Japan), etc. To facilitate the subsequent cloning operation, it is preferable to add appropriate restriction sites to primers.

SEQ ID NOS: 1, 3, 5 and 7 have a nucleotide sequence GGAGGTTGTTTT, which contains the SD sequence GGAGG of a penP gene (FEBS Letters 422, 243, (1998)) behind the restriction site. By adding this SD sequence, it becomes possible to allow the gene of Streptomyces to be expressed in *Bacillus subtilis.*

Examples of the reaction conditions for PCR include a condition that a reaction process of 94°C to 95°C, 30 seconds (denaturation), 55°C to 60°C, 30 seconds to 1 minute (annealing) and around 72°C, 1 to 2 minutes (extension) is defined as 1 cycle, and this process is repeated, for example, for 20 to 40 cycles, and then a reaction is carried out at 72°C for 7 to 10 minutes. A DNA fragment can be cloned in an appropriate vector which can be amplified in *E. coli.* However, conditions for PCR are not limited to the above-described conditions, and changes and modifications can be made by a person skilled in the art as appropriate.

The cloning of the amplified DNA can be carried out by a conventional method such as one described in Molecular Cloning 2^{nd} Ed., Current Protocols in Molecular Biology, Supplement 1-38, John Wiley & Sons (1987-1997) (hereinafter, abbreviated as Current Protocols in Molecular Biology), DNA Cloning 1: Core Techniques, A Practical Approach, Second Edition, Oxford University Press (1995), etc. or by using a commercially available kit such as Superscript Plasmid System for cDNA Synthesis and Plasmid Cloning (Life Technology) or ZAP-cDNA Synthesis Kit (Staratagene).

Any vector such as phage vector or plasmid vector can be used as a cloning vector, as long as it can autonomously replicate in *E*. *coli* K12. An *E*. *coli* expression vector may be used as a cloning vector. Specific examples include ZAP Express [Stratagene, Strategies, 5, 58 (1992)], pBluescript II SK(+) [Nucleic Acids Research, 17, 9494(1989)], Lambda ZAP II (Stratagene), λ gt10, λ gt11 [DNA Cloning, A Practical Approach, 1, 49(1985)], λ TriplEx (Clonetech), λ ExCell (Pharmacia), pT7T318U (Pharmacia), pcD2 [Mol. Cen. Biol., 3, 280 (1983)], pMW218 (Wako Pure Chemical Industries, Ltd.), pUC118 (Takara Shuzo Co., Ltd.), pEG400 [J.Bac., 172, 2392 (1990)], and pQE-30 (QIAGEN).

A plasmid containing DNA of interest can be obtained from the obtained transformant according to a conventional method such as one described in Molecular Cloning 2^{nd} Ed., Current Protocols in Molecular Biology, DNA Cloning 1: Core Techniques, A Practical Approach, Second Edition, Oxford University Press (1995), etc. By the above-described method, DNA having a nucleotide sequence of SEQ ID NOS: 9, 10, 11 and 12 can be obtained.

Moreover, in the present invention, there can also be used DNA which has a deletion, substitution, addition and/or insertion of one or several nucleotides in the DNA having the nucleotide sequence of SEQ ID NOS: 9 to 12, and can allow the mevalonate pathway to function in the presence of mevalonic acid; or DNA which can hybridize with the DNA having the nucleotide sequence of SEQ ID NOS: 9 to 12 under stringent condition, and can allow the mevalonate pathway to function in the presence of mevalonic acid.

Such DNA can be isolated by using the nucleotide sequence of a DNA fragment derived from Streptomyces having the nucleotide sequence of SEQ ID NOS: 9 to 12 and screening for the homolog of the DNA from other microorganisms or the like under appropriate condition, or it can be produced by a method known to a person skilled in the art such as chemical synthesis, genetic engineering or mutagenesis.

For example, such DNA can be obtained by a method of contacting DNA having the nucleotide sequence of SEQ ID NOS: 9 to 12 with an agent of mutagene, a method of applying ultraviolet ray to the DNA, a method of genetic engineering technique, or the like. The site-directed mutagenesis which is one of the genetic engineering technique is useful as a technique for introducing a specific mutation into a specific position, and can be carried out according to a method described in Molecular Cloning 2^{nd} Ed.; Current Protocols in Molecular Biology; Nucleic Acids Research, 10, 6487, 1982; Nucleic Acids Research, 12, 9441, 1984; Nucleic Acids Research, 13, 4431, 1985; Nucleic Acids Research, 13, 8749, 1985; Proc. Natl. Acad. Sci. USA, 79, 6409, 1982; Proc. Natl. Acad. Sci. USA, 82, 488, 1985; Gene, 34, 315, 1985; Gene, 102, 67, 1991; etc.

To allow the thus obtained DNA to be expressed in a host cell, the DNA fragment of interest is first cleaved with a restriction enzyme or deoxyribonuclease into the appropriate length of a DNA fragment comprising the gene. The obtained DNA fragment is inserted downstream of a promoter in an expression vector, and then the expression vector into which the above DNA is inserted is introduced into a host cell suitable for the expression vector.

The DNA fragments encoding phosphomevalonate kinase (SEQ ID NO: 9), diphosphomevalonate decarboxylase (SEQ ID NO: 10), mevalonate kinase (SEQ ID NO: 11) and IPP isomerase (SEQ ID NO: 12) may be incorporated into expression vectors separately, and then the obtained 4 types of expression vectors may be introduced into a host. Otherwise, 2 types or more, preferably 3 types or more, and more preferably all the 4 types of the above DNA fragments may be cloned into a single expression vector and then this expression vector may be introduced into a host. From the viewpoint of operability, it is preferable that an expression vector into which the above 4 types of DNA fragments are incorporated is introduced into a host.

The type of a host is not particularly limited, as long as it has the non-mevalonate pathway. Preferred type of a host is a bacterium or a plant cell. Examples of a bacterium include a Gram-positive bacterium *(Micrococcus* sp., *Bacillus* sp., *Clostridium* sp., *Corynebacterium* sp., etc.), and a Gram-negative bacterium (*Rhodopseudomonas* sp., *Pseudomonas* sp., *Escherichia* sp., *Salmonella* sp., *Thiobacillus* sp., *Neisseria* sp., etc.). Since a Gram-positive bacterium has a higher efficiency of taking a substance into a cell than a Gram-negative bacterium, the Gram-positive bacterium is preferable.

As an expression vector, there is used a vector which can autonomously replicate in the above host cell or can be incorporated into a chromosome and which comprises a promoter in a position where the above DNA of interest can be transcribed.

Where a bacterium is used as a host cell, the expression vector for expressing the above DNA is capable of autonomous replication in the bacterium, and also is preferred to be a recombinant vector composed of a promoter, a ribosome binding sequence, the above DNA and a transcription termination sequence. A gene which regulates the promoter may also be contained therein.

Examples of an expression vector include pAA101 (Amersham), pBTrP2, pBTac1, pBTac2 (all of these are commercially available from Boehringer Mannheim), pKK233-2 (Pharmacia), pSE280 (Invitrogen), pGEMEX-1 (Promega), pQE-8 (QIAGEN), pQE-30 (QIAGEN), pKYP10 (Japanese Patent Application Laying-Open (Kokai) No. 58-110600), pKYP200 [Agrc. Biol. Chem., 48, 669 (1984)], PLSA1 [Agrc. Biol. Chem., 53, 277 (1989)], pGEL1 [Proc. Natl. Acad. Sci. USA, 82, 4306 (1985)], pBluescriptII SK+, pBluescriptII SK(-) (Stratagene), pTrS30 (FERMBP-5407), pTrS32 (FERM BP-5408), pGEX (Pharmacia), pET-3 (Novagen), pTerm2 (US4686191, US4939094, US5160735), pSupex, pUB110, pTP5, pC194, pUC18 [Gene, 33, 103 (1985)], pUC19 [Gene, 33, 103 (1985)], pSTV28 (Takara Shuzo Co., Ltd.), pSTV29 (Takara Shuzo Co., Ltd.), pUC118 (Takara Shuzo Co., Ltd.), pPA1 (Japanese Patent Application Laying-Open (Kokai) No. 63-233798), pEG400 [J. Bacteriol., 172, 2392 (1990)], and pQE-30 (QIAGEN).

Any promoter can be used, as long as expression can be carried out in a host cell. Examples of a promoter include a promoter derived from *Escherichia coli* or phage, such as a trp promoter (P trp), lac promoter (P lac), P_{L} promoter, P_{R} promoter or P_{SE} promoter, SP01 promoter, SP02 promoter, and penP promoter. Moreover, artificially designed or modified promoters such as a promoter obtained by connecting two P trp promoters in series (P trp x 2), tac promoter, letl promoter and lacT7 promoter, can also be used.

As a method of introducing a recombinant vector, any method can be applied as long as it is a method of introducing DNA into the above host cell. Examples of such a method include a method using calcium ion [Proc. Natl. Acad. Sci. USA, 69, 2110 (1972)], the protoplast method (Japanese Patent Application Laying-Open (Kokai) No. 63-2483942), and a method described in Gene, 17, 107 (1982) or Molecular & General Genetics, 168, 111 (1979).

The thus produced transformant of the present invention can be cultured in a medium according to a common method used for the culture of a host.

The medium may be either a natural medium or a synthetic medium, as long as the medium contains a carbon source, a nitrogen source, inorganic salts or the like that can be assimilated by the transformant of the present invention, and enables efficient culturing of the transformant.

Any carbon source may be used, as long as it can be assimilated by each microorganism. Examples thereof include carbohydrate such as glucose, fructose, sucrose, molasses containing them, starch or starch hydrolysate; organic acid such as acetic acid or propionic acid; and alcohols such as ethanol and propanol. Examples of a nitrogen source include ammonia; ammonium salts of various inorganic or organic acids such as ammonium chloride, ammonium sulfate, ammonium acetate and ammonium phosphate; other nitrogen-containing compounds; and peptone, meat extract, yeast extract, corn steep liquor, casein hydrolysate, soybean cake and soybean cake hydrolysate, various fermentative cell bodies and digests thereof; etc. Examples of an inorganic product include potassium monophosphate, potassium diphosphate, magnesium phosphate, magnesium sulfate, sodium chloride, ferrous sulfate, manganese sulfate, copper sulfate, and calcium carbonate.

Culturing can be carried out under aerobic conditions such as shaking culturing or deep aeration and agitation culturing. The culturing temperature is preferably 15°C to 40°C, and the culturing period is generally 16 hours to 7 days. The pH in culturing is retained at 3.0 to 9.0. The control of pH is carried out using inorganic or organic acid, an alkali solution, urea, calcium carbonate, ammonia, etc.

In addition, antibiotics such as ampicillin or tetracycline may be added to a medium during culturing as necessary.

When a microorganism transformed with an expression vector comprising an inductive promoter as a promoter is cultured, an inducer may be added to a medium as necessary. For example, when a microorganism transformed with an expression vector comprising a lac promoter is cultured, isopropyl- β -D-thiogalactopyranoside (IPTG) or the like may be added to a medium, and when a microorganism transformed with an expression vector comprising a trp promoter is cultured, indoleacrylic acid (IAA) or the like may be added thereto.

### (C) Method for screening substances specifically inhibiting the non-mevalonate pathway by using an organism capable of using both the mevalonate pathway and the non-mevalonate pathway as biosynthetic pathways of isopentenyl diphosphate (IPP) and dimethylallyl diphosphate (DMAPP)

An organism capable of using both the mevalonate pathway and the non-mevalonate pathway which is produced in (B) above is cultured with a test substance under both an environment where the mevalonate pathway can be used and an environment where the mevalonate pathway cannot be used, and then a test substance which inhibits the growth of the organism under an environment where the mevalonate pathway cannot be used, but does not inhibit the growth of the organism under an environment where the mevalonate pathway can be used, is selected. By this method, a substance having an activity of specifically inhibiting the non-mevalonate pathway can be identified.

Embodiments of culturing an organism with a test substance under an environment where the mevalonate pathway can be used and under an environment where the pathway cannot be used, include, for example, the following two types.

In the first embodiment, using an organism having enzymes which catalyze the reaction after mevalonic acid on the mevalonate pathway, the organism is cultured with a test substance both in a medium containing mevalonic acid (an environment where the mevalonate pathway can be used) and in a medium containing no mevalonic acid (an environment where the mevalonate pathway cannot be used).

In the second embodiment, two types of organisms, that is, an organism having all enzymes which catalyze all processes of converting acetoacetyl CoA into isopentenyl pyrophosphate (IPP) and dimethylallyl diphosphate (DMAPP) by way of HMG-CoA, mevalonic acid and pyrophosphomevalonic acid (i.e. an organism capable of using the mevalonate pathway in the absence of mevalonic acid), and an organism incapable of using the mevalonate pathway, are cultured with a test substance in the same medium.

In the present invention, the above-described first embodiment is particularly preferable.

Moreover, in the present invention, the above screening method can be carried out using an organism wherein IPP isomerase gene is destructed. By performing screening using such a mutant organism, it also becomes possible to screen inhibitors which are specific for the reaction of synthesizing isopentenyl diphosphate (IPP) or dimethylallyl diphosphate (DMAPP) from an intermediate substance on the non-mevalonate pathway. That is to say, a transformant is prepared by introducing DNA fragments encoding enzymes existing on the mevalonate pathway, i.e., phosphomevalonate kinase, diphosphomevalonate decarboxylase, mevalonate kinase and IPP isomerase, into an organism wherein IPP isomerase gene is destructed. This transformant is then allowed to grow in the presence of a test substance under the two conditions of: (1) mevalonic acid is absent and the introduced IPP isomerase gene is not expressed; and (2) mevalonic acid is present and all of the introduced genes of the enzymes on the mevalonate pathway are expressed. By selecting a test substance with which the transformant cannot grow under the conditions of (1) above but can grow under the conditions of (2) above, a substance which specifically inhibits any enzyme involved in the non-mevalonate pathway leading to IPP or DMAPP can be screened.

Fosmidomycin is an inhibitor which is specific for DXP reductoisomerase that is an enzyme at the second stage on the non-mevalonate pathway (Tetrahedron Lett. 39, 7913, (1998); Science 285, 1573, (1999)), and is a compound having an anti-bacterial activity (J. Antimicrob. Chemother. 24, 131, (1989)), a herbicide activity (US Patent No. 4,693,742) or an anti-malarial activity (Science 285, 1573, (1999)). In the examples of the present specification set forth later, the growth of *Bacillus subtilis* has been analyzed by culturing *Bacillus subtilis,* which can use the mevalonate pathway in the presence of mevalonic acid and IPTG and also can use the non-mevalonate pathway, together with fosmidomycin (a test substance) in the presence of mevalonic acid and IPTG (i.e. under an environment where the mevalonate pathway can be used) or in the absence of mevalonic acid and IPTG (i.e. under an environment where the mevalonate pathway cannot be used). As a result, it was confirmed that fosmidomycin does not show an anti-bacterial activity in the presence of mevalonic acid and IPTG, but shows an anti-bacterial activity in the absence of mevalonic acid and IPTG. From this result, it was demonstrated that the screening method of the present invention was useful for screening substances which specifically inhibit the non-mevalonate pathway.

The screening method of the present invention is suitable for examining various kinds of compounds for a substance which is likely to specifically inhibit the non-mevalonate pathway, and the type of the test substance is not particularly limited. Where the first screening is carried out on test substances using the screening method of the present invention, various compound libraries can be used. Examples of such compound libraries include a combinatorial library, a natural product library, other small molecule compound libraries, etc. Moreover, a commercially available compound from a commercial source can also be screened.

Examples of a test substance include a low molecular weight compound, and peptide.

The present invention will be explained further in detail below, but the present invention is not limited by the examples.

### Examples

### Example 1: Obtainment of DNAs encoding proteins involved in the mevalonate pathway which functions in Bacillus subtilis

According to a conventional method, chromosomal DNA was isolated and purified from *Streptomyces* sp. CL190. Sense primers and antisense primers having each of the combinations of the nucleotide sequences of SEQ ID NOS: 1 and 2, SEQ ID NOS: 3 and 4, SEQ ID NOS: 5 and 6, and SEQ ID NOS: 7 and 8, were synthesized using a DNA synthesizer.

Using the sense primers and the antisense primers having each of the combinations of the nucleotide sequences of SEQ ID NOS: 1 and 2, SEQ ID NOS: 3 and 4, SEQ ID NOS: 5 and 6, and SEQ ID NOS: 7 and 8 separately, the following PCR is carried out using the chromosomal DNA of the CL190 strain as a template, so as to amplify DNA fragments each encoding each of phosphomevalonate kinase (SEQ ID NO: 9), diphosphomevalonate decarboxylase (SEQ ID NO: 10), mevalonate kinase (SEQ ID NO: 11) and IPP isomerase (SEQ ID NO: 12).

Moreover, as a restriction site, SEQ ID NO: 1 has HindIII at the 5'-terminus, SEQ ID NOS: 2 and 3 have XbaI at the 5'-terminus, SEQ ID NOS: 4 and 5 have BgIII at the 5'-terminus, SEQ ID NOS: 6 and 7 have ClaI at the 5'-terminus, and SEQ ID NO: 8 has SphI at the 5'-terminus.

Furthermore, SEQ ID NOS: 1, 3, 5 and 7 have a nucleotide sequence, GGAGGTTGTTTT, which comprises the SD sequence GGAGG of penP gene (FEBS Letters 422, 243, (1998)) behind the restriction site. By adding this SD sequence, the gene of Streptomyces can be expressed in *Bacillus subtilis.*

Using chromosomal DNA as a template, PCR was carried out using these primers and Expand^{™} High-Fidelity PCR System (Boehringer Mannheim). PCR was carried out by conducting reaction at 95°C for 1 minute, repeating the cycles of 95°C for 30 seconds, 60°C for 30 seconds and 72°C for 1 minute 30 times, and then conducting reaction 72°C for 10 minutes.

Each DNA fragment amplified by PCR was obtained by agarose gel electrophoresis, and after purification, the purified DNA fragment was dissolved in 5 *µ*l of TE buffer.

To these solutions was added 0.5 µl of pGEM T-easy vector (Promega), and then ligation reaction was carried out to obtain recombinant DNAs.

Using this recombinant DNAs, *Escherichia coli* JM109 was transformed by a conventional method, and then the obtained transformants were applied on an LB agar medium containing 50 µg/ml ampicillin followed by culturing at 37°C overnight.

Several colonies of the growing ampicillin-resistant transformants were subjected to shaking culturing at 37°C overnight in 5 ml of LB liquid medium containing 50 µg/ml ampicillin.

The obtained culture solutions were centrifuged to obtain cells.

Plasmids were isolated from the obtained cells according to a conventional method.

The plasmids which were isolated by the above method were cleaved with various types of restriction enzymes to examine the structures and the nucleotide sequences were determined, and it was thus confirmed that these were plasmids into which the DNA fragments having correct nucleotide sequences of interest were inserted.

A plasmid containing DNA having the nucleotide sequence of SEQ ID NO: 9 was named as pGEMMVK1, a plasmid containing DNA having the nucleotide sequence of SEQ ID NO: 10 was named as pGEMMDC, a plasmid containing DNA having the nucleotide sequence of SEQ ID NO: 11 was named as pGEMMVK2, and a plasmid containing DNA having the nucleotide sequence of SEQ ID NO: 12 was named as pGEMHYP.

The pGEMMVK2 was treated with BgIII and ClaI, and a fragment having the nucleotide sequence of SEQ ID NO: 11 was obtained by agarose gel electrophoresis, and was then subcloned into an expression vector pAA101 (Amersham) by a conventional method. The plasmid obtained by this subcloning was named as pBMV1.

The pGEMHYP was treated with ClaI and SphI, and a fragment having the nucleotide sequence of SED ID NO: 12 was obtained by agarose gel electrophoresis, and was then subcloned into the pBMV1 by a conventional method. The plasmid obtained by this subcloning was named as pBMV2.

The pGEMMDC was treated with XbaI and BglII, and a fragment having the nucleotide sequence of SEQ ID NO: 10 was obtained by agarose gel electrophoresis, and was then subcloned into the pBMV2 according to a conventional method. The plasmid obtained by this subcloning was named as pBMV3.

The pGEMMVK1 was treated with HindIII and XbaI, and then subcloned into the pBMV3 according to a conventional method. The plasmid obtained by this subcloning was named as pBMV4.

### Example 2: Screening of an inhibitor on the non-mevalonate pathway

According to a conventional method, the pBMV4 was introduced into *B*. *subtilis* 168 strain to obtain a transformant, *B. subtilis* 168 (pBMV4) which shows resistance to chloramphenicol with a concentration of 3.4 µg/ml. The *B. subtilis* 168 (pBMV4) was subjected to shaking culturing at 37°C overnight in 5 ml of LB liquid medium containing 3.4 µg/ml chloramphenicol.

One thousandth amount of the above culture solution was added into Nutrient Broth "Eiken" (Eiken Chemical Co., Ltd.) containing 1mM IPTG and 3.4 µg/ml chloramphenicol to prepare an agar medium.

One thousandth amount of the above culture solution was added into Nutrient Broth "Eiken" containing 1mM IPTG, 3.4 µg/ml chloramphenicol and 0.02% mevalonic acid to prepare an agar medium. The gene on the pBMV4 is expressed only in the presence of IPTG.

50 µl of aqueous solution of 5 µg/ml fosmidomycin was dropped on a paper disk, and then the paper disk was placed on the above two types of agar mediums, followed by culturing at 37°C overnight.

After completion of the culturing, a growth inhibition ring due to fosmidomycin was not observed in a medium to which mevalonic acid and IPTG were added, whereas a growth inhibition ring due to fosmidomycin was observed in a medium to which mevalonic acid and IPTG were not added. The inhibition of the non-mevalonate pathway due to fosmidomycin could be confirmed by using media with or without mevalonic acid and IPTG, and *B. subtilis* 168 (pBMV4).

According to the similar method, by using a test compound instead of fosmidomycin, a substance which inhibits any enzyme reaction on the non-mevalonate pathway can be screened.

### Example 3: Construction of IPP isomerase gene (ypgA) disrupted strain of Bacillus subtilis

The ypgA gene of *Bacillus subtilis* encodes IPP isomerase (Proc. Natl. Acad. Sici. USA., 98, 932-937, 2001).

Using the following primers which are specific for the ypgA gene:
N-terminus primer (SEQ ID NO: 14) GGGGATCCTTGGAGCAAACGCACTGC
C-terminus primer (SEQ ID NO: 15) GGGGATCCAAAAAAGGAGATTTGCCGC
a partial region of the ypgA gene was amplified by PCR. Using these primers, the sequence corresponding to nucleotides 416 to 693 of the ypgA gene (1,057 nucleotides) can be amplified. After confirmation of the nucleotide sequence, this amplified DNA fragment was ligated to the BamHI site of a vector for *Bacillus subtilis*, pMUTIN2 (MICROBIOLOGY, 144: 3097-3104, 1998), and the constructed plasmid was named as pMYPGA.

The *B. subtilis* 168 was transformed with the pMYPGA, and an erythromycin-resistant clone was selected. In the erythromycin-resistant clone, it is predicted that the ypgA gene of the genome is destructed as a result of homologous recombination. It was confirmed by Southern hybridization that the ypgA gene of the obtained erythromycin-resistant clone was destructed. This IPP isomerase gene (ypgA) disrupted strain was named as *B. subtilis* delypgA.

### Example 4: Screening of inhibitors on the non-mevalonate pathway

According to a conventional method, the pBMV4 was introduced into the *B. subtilis* delypgA strain produced in Example 3 so as to obtain a transformant *B. subtilis* delypgA (pBMV4) which shows resistance to chloramphenicol with a concentration of 3.4 µg/ml. The *B. subtilis* delypgA (pBMV4) was subjected to shaking culturing at 37°C overnight in 5 ml of LB liquid medium containing 3.4 *µ* g/ml chloramphenicol.

One thousandth amount of the above culture solution was added into Nutrient Broth "Eiken" (Eiken Chemical Co., Ltd.) containing 3.4 µg/ml chloramphenicol to prepare an agar medium.

One thousandth amount of the above culture solution was added into Nutrient Broth "Eiken" containing 1mM IPTG, 3.4 µg/ml chloramphenicol and 0.02% mevalonic acid to prepare an agar medium. The gene on the pBMV4 is expressed only in the presence of IPTG

50 µl of aqueous solution of 5 µg/ml fosmidomycin was dropped on a paper disk, and then the paper disk was placed on the above two types of agar mediums, followed by culturing at 37°C overnight.

After completion of the culture, a growth inhibition ring due to fosmidomycin was not observed in a medium to which mevalonic acid and IPTG were added, whereas the growth inhibition ring due to fosmidomycin was observed in a medium to which mevalonic acid and IPTG were not added. The inhibition of the non-mevalonate pathway due to fosmidomycin could be confirmed by using media with or without mevalonic acid and IPTG, and *B. subtilis* delypgA (pBMV4).

According to the similar method, by using a test compound instead of fosmidomycin, substances which inhibit any enzyme reaction on the non-mevalonate pathway can be screened.

### Industrial Applicability

According to the present invention, it is possible to provide a novel method for screening anti-bacterial agents, herbicides or anti-malarial agents which can specifically inhibit any of enzyme reactions on the non-mevalonate pathway for synthesizing isopentenyl diphosphate. In the present invention, operations are simple and screening efficiency is high, since it is not necessary to purify an enzyme involved in the non-mevalonate pathway and determine its enzyme activity. Moreover, anti-bacterial agents, herbicides or anti-malarial agents which can specifically inhibit an unknown enzyme reaction on the non-mevalonate pathway can also be screened by the screening method of the present invention.

## Claims

1. A method for screening substances which specifically inhibit the non-mevalonate pathway by using a non-human organism capable of using both the mevalonate pathway and the non-mevalonate pathway as biosynthetic pathways of isopentenyl diphosphate (IPP) and dimethylallyl diphosphate (DMAPP),
wherein said non-human organism is capable of using the mevalonate pathway only in the presence of mevalonic acid, wherein the non-human organism is a transformant obtained by introducing DNA encoding all enzymes necessary for using the mevalonate pathway in the presence of mevalonic acid, into a non-human organism having the non-mevalonate pathway, wherein said enzymes are at least phosphomevalonate kinase, diphosphomevalonate decarboxylase, mevalonate kinase and IPP isomerase, and
wherein the non-human organism is cultured with a test substance in the presence or absence of mevalonic acid, and wherein a test substance which does not inhibit the growth of the non-human organism in the presence of mevalonic acid but inhibits the growth of the non-human organism in the absence of mevalonic acid is indicating that said test substance inhibits specifically the non-mevalonate pathway and said test substance is selected as a candidate substance.

2. The method of claim 1, wherein the DNA encoding the enzymes necessary for using the mevalonate pathway in the presence of mevalonic acid is any of the followings:
(1) DNA having all nucleotide sequences of SEQ ID NOS: 9 to 12;
(2) DNA which has a deletion, substitution, addition and/or insertion of one or several nucleotides in the DNA of (1) above, and can allow the mevalonate pathway to function in the presence of mevalonic acid; or
(3) DNA which can hybridize with the DNA of (1) above under stringent condition, and can allow the mevalonate pathway to function in the presence of mevalonic acid.

3. A method for screening substances which specifically inhibit the non-mevalonate pathway by using a non-human organism capable of using both the mevalonate pathway and the non-mevalonate pathway as biosynthetic pathways of isopentenyl diphosphate (IPP) and dimethylallyl diphosphate (DMAPP), wherein the non-human organism capable of using both the mevalonate pathway and the non-mevalonate pathway is a non-human organism capable of using the mevalonate pathway in the absence of mevalonic acid, and wherein said non-human organism is a transformant obtained by introducing DNA encoding all enzymes necessary for using the mevalonate pathway in the absence of mevalonic acid, into a non-human organism having the non-mevalonate pathway, wherein said enzymes are at least HMG-CoA synthase, HMG-CoA reductase, phosphomevalonate kinase, diphosphomevalonate decarboxylase, mevalonate kinase and IPP isomerase, and
wherein the non-human organism capable of using both the mevalonate pathway and the non-mevalonate pathway and a non-human organism incapable of using the mevalonate pathway but capable of using the non-mevalonate pathway are each cultured with a test substance, and wherein said test substance which does not inhibit the growth of the non-human organism capable of using both the mevalonic pathway and the non-mevalonic pathway but inhibits the growth of the non-human organism incapable of using the mevalonate pathway but capable of using the non-mevalonate pathway is indicating that said test substance inhibits specifically the non-mevalonate pathway and said test substance is selected as candidate substance.

4. The method of claim 3, wherein the DNA encoding the enzymes necessary for using the mevalonate pathway in the absence of mevalonic acid is any of the followings:
(1) DNA having a nucleotide sequence of SEQ ID NO: 13;
(2) DNA which has a deletion, substitution, addition and/or insertion of one or several nucleotides in the nucleotide sequence of SEQ ID NO: 13, and can allow the mevalonate pathway to function in the absence of mevalonic acid; or
(3) DNA which can hybridize with the DNA of (1) above under stringent condition, and can allow the mevalonate pathway to function in the absence of mevalonic acid.

5. The method of any one of claims 1 to 4, wherein the non-human organism not having IPP isomerase is used as said non-human organism.

6. The method of any one of claims 1 to 5, wherein the organism is a bacterium.

7. The method of any one of claims 1 to 6, wherein the organism is a Gram-positive bacterium.

8. The method of any of claims 1 to 7, wherein the organism is *Bacillus subtilis.*

9. The method of any one of claims 1 to 8 which is used for the sreening of candidate compounds for anti-bacterial agents, herbicides or anti-malarial agents.

10. A non-human transformant which is obtained by introducing DNA encoding all enzymes necessary for using the mevalonate pathway as a biosynthetic pathway of isopentenyl diphosphate (IPP) and dimethylallyl diphosphate (DMAPP) in the presence of mevalonic acid, into a non-human organism having the non-mevalonic pathway only, wherein said enzymes are at least phosphomevalonate kinase, diphosphomevalonate decarboxylase, mevalonate kinase and IPP isomerase.

11. The transformant of claim 10, wherein the DNA encoding the enzymes necessary for using the mevalonate pathway in the presence of mevalonic acid is any of the followings:
(1) DNA having all nucleotide sequences of SEQ ID NOS: 9 to 12;
(2) DNA which has a deletion, substitution, addition and/or insertion of one or several nucleotides in the DNA of (1) above, and can allow the mevalonate pathway to function in the presence of mevalonic acid; or
(3) DNA which can hybridize with the DNA of (1) above under stringent condition, and can allow the mevalonate pathway to function in the presence of mevalonic acid.

## Patentansprüche

1. Ein Verfahren zum Screenen von Substanzen, die spezifisch den Nicht-Mevalonat Biosyntheseweg hemmen, unter Verwendung eines nicht menschlichen Organismus, der beide Biosynthesewege, den Mevalonat Biosyntheseweg und den Nicht-Mevalonat Biosyntheseweg, als Biosynthesewege von Isopentenyl diphosphat (IPP) und Dimethylallyl diphosphat (DMAPP) nutzen kann,
wobei der nicht menschliche Organismus nur in Anwesentheit von Mevalonsäure in der Lage ist den Mevalonat Biosyntheseweg zu nutzen,
wobei der nicht menschliche Organismus ein Transformant ist, der durch Einführen einer DNA, die für alle Enzyme codiert, die für die Verwendung des Mevalonat Biosynthesewegs in Anwesenheit von Mevalonsäure notwendig sind, in einen nicht menschlichen Organismus erhalten wurde, der den Nicht-Mevalonat Biosyntheseweg aufweist, wobei die Enzyme zumindest Phosphomevalonatkinase, Diphosphomevalonat-decarboxylase, Mevalonatkinase und IPP Isomerase sind, und
wobei der nicht menschliche Organismus mit einer Testsubstanz in Anwesenheit oder Abwesenheit von Mevalonsäure kultiviert wird, und
wobei eine Testsubstanz, die in Anwesenheit von Mevalonsäure das Wachstum des nicht menschlichen Organismus nicht hemmt, aber die in Abwesenheit von Mevalonsäure das Wachstum des nicht menschlichen Organismus hemmt, anzeigt, dass die Testsubstanz den Nicht-Mevalonat Biosyntheseweg spezifisch hemmt; und wobei diese Testsubstanz als Anwärtersubstanz ausgewählt wird.

2. Das Verfahren nach Anspruch 1, wobei die DNA, die für die Enzyme codiert, die für die Verwendung des Mevalonat Biosynthesewegs in Anwesenheit von Mevalonsäure notwendig sind, irgend eine der folgenden ist:
(1) eine DNA mit allen Nukleotidsequenzen SEQ ID NO: 9 bis 12;
(2) eine DNA, die eine Deletion, Substitution, Addition und/oder eine Insertion von einem oder mehreren Nukleotiden in der DNA gemäß (1) aufweist, und die Funktion des Mevalonat Biosyntheseweges in Anwesenheit von Mevalonsäure erlaubt; oder
(3) eine DNA, die mit der DNA gemäß (1) unter stringenten Bedingungen hybridisieren kann, und die Funktion des Mevalonat Biosyntheseweges in Anwesenheit von Mevalonsäure erlaubt.

3. Ein Verfahren zum Screenen von Substanzen, die spezifisch den Nicht-Mevalonat Biosyntheseweg hemmen, unter Verwendung eines nicht menschlichen Organismus, der beide Biosynthesewege, den Mevalonat Biosyntheseweg und den Nicht-Mevalonat Biosyntheseweg, als Biosynthesewege von Isopentenyl diphosphat (IPP) und Dimethylallyl diphosphat (DMAPP) nutzen kann, wobei der nicht menschliche Organismus, der in der Lage ist beide Biosynthesewege, den Mevalonat Biosyntheseweg und den Nicht-Mevalonat Biosyntheseweg, zu nutzen, ein nicht menschlicher Organismus ist, der in der Lage ist den Mevalonat Biosyntheseweg in Abwesenheit von Mevalonsäure zu nutzen, und wobei der nicht menschliche Organismus ein Transformant ist, der durch Einführen einer DNA erhalten wurde, die für alle Enzyme codiert, die für die Verwendung des Mevalonat Biosyntheseweges in Abwesenheit von Mevalonsäure notwendig sind, in einen nicht menschlichen Organismus, der den nicht Mevalonat Biosyntheseweg aufweist, wobei die Enzyme zumindest HMG-CoA Synthase, HMG-CoA Reduktase, Phosphomevalonatkinase, Diphosphomevalonatdecarboxylase, Mevalonatkinase und IPP Isomerase sind, und
wobei der nicht menschliche Organismus, der in der Lage ist beide Biosynthesewege zu nutzen, den Mevalonat Biosyntheseweg und den Nicht-Mevalonat Biosyntheseweg, und ein nicht menschlicher Organismus, der nicht in der Lage ist den Mevalonat Biosyntheseweg zu nutzen, aber in der Lage ist den Nicht-Mevalonat Biosyntheseweg zu nutzen, gemeinsam mit einer Testsubstanz kultiviert werden, und wobei eine Testsubstanz, die das Wachstum des nicht menschlichen Organismus, der in der Lage ist beide Biosynthesewege zu nutzen, den Mevalonat Biosyntheseweg und den Nicht-Mevalonat Biosynthesweg, nicht hemmt, aber das Wachstum des nicht menschlichen Organismus, der nicht in der Lage ist den Mevalonat Biosynthesweg zu nutzen, aber in der Lage ist den Nicht-Mevalonat Biosyntheseweg zu nutzen, hemmt, anzeigt, dass die Testsubstanz den Nicht-Mevalonat Biosyntheseweg spezifisch hemmt; und
wobei diese Testsubstanz als Anwärtersubstanz ausgewählt wird.

4. Das Verfahren nach Anspruch 3, wobei die DNA, die für die Enzyme codiert, die für die Verwendung des Mevalonat Biosynthesweg in Abwesenheit von Mevalonsäure notwendig sind, irgend eine der folgenden ist:
(1) eine DNA, die eine Nukleotidsequenz mit der SEQ ID NO: 13 aufweist;
(2) eine DNA, die eine Deletion, Substitution, Addition und/oder Insertion von einem oder mehreren Nukleotiden in der Nukleotidsequenz der SEQ ID NO: 13 aufweist, und die Funktion des Mevalonat Biosyntheseweges in Abwesenheit von Mevalonsäure erlaubt; oder
(3) eine DNA, die mit der DNA gemäß (1) unter stringenten Bedingungen hybridisieren kann, und die Funktion des Mevalonat Biosynthesweges in Abwesenheit von Mevalonsäure erlaubt.

5. Das Verfahren nach irgend einem der Ansprüche 1 bis 4, wobei der nicht menschliche Organismus, der die IPP Isomerase nicht aufweist, als nicht menschlicher Organismus verwendet wird.

6. Das Verfahren nach irgend einem der Ansprüche 1 bis 5, wobei der Organismus ein Bakterium ist.

7. Das Verfahren nach irgend einem der Ansprüche 1 bis 6, wobei der Organismus ein Gram-positives Bakterium ist.

8. Das Verfahren nach irgend einem der Ansprüche 1 bis 7, wobei der Organismus *Bacillus subtilis* ist.

9. Das Verfahren nach irgend einem der Ansprüche 1 bis 8, das verwendet wird zum Screenen von Anwärtersubstanzen für antibakterielle Wirkstoffe, Herbizide oder Anti-Malaria-Mittel.

10. Ein nicht menschlicher Transformant, der durch Einführen einer DNA erhalten wurde, die für alle Enzyme codiert, die für die Verwendung des Mevalonat Biosynthesweges als Biosynthesweg für Isopentenyldiphosphat (IPP) und Dimethylallyldiphosphat (DMAPP) in Anwesenheit von Mevalonsäure notwendig sind, in einen nicht menschlichen Organismus, der nur den Nicht-Mevalonat Biosynthesweg aufweist, wobei die Enzyme zumindest Phosphomevalonatkinase, Diphosphomevalonatdecarboxylase, Mevalonatkinase und IPP Isomerase sind.

11. Der Transformant gemäß Anspruch 10, wobei die DNA, die für die Enzyme codiert, die für die Verwendung des Mevalonat Biosynthesewegs in Anwesenheit von Mevalonsäure notwendig sind, irgend eine der folgenden ist:
(1) eine DNA mit allen Nukleotidsequenzen SEQ ID NO: 9 bis 12;
(2) eine DNA, die eine Deletion, Substitution, Addition und/oder eine Insertion von einem oder mehreren Nukleotiden in der DNA gemäß (1) aufweist, und die Funktion des Mevalonat Biosyntheseweges in Anwesenheit von Mevalonsäure erlaubt; oder
(3) eine DNA, die mit der DNA gemäß (1) unter stringenten Bedingungen hybridisieren kann, und die Funktion des Mevalonat Biosyntheseweges in Anwesenheit von Mevalonsäure erlaubt.

## Revendications

1. Procédé pour cribler des substances qui inhibent spécifiquement la voie non mévalonate en utilisant un organisme non humain capable d'utiliser à la fois la voie mévalonate et la voie non mévalonate en tant que voies biosynthétiques de l'isopentényl diphosphate (IPP) et du diméthylallyl diphosphate (DMAPP),
dans lequel ledit organisme non humain est capable d'utiliser la voie mévalonate uniquement en présence d'acide mévalonique, où l'organisme non humain est un transformant obtenu par l'introduction d'un ADN codant pour toutes les enzymes nécessaires pour utiliser la voie mévalonate en présence d'acide mévalonique, à l'intérieur d'un organisme non humain possédant la voie non mévalonate, où lesdites enzymes sont au moins phosphomévalonate kinase, diphosphomévalonate décarboxylase, mévalonate kinase et IPP isomérase, et
dans lequel l'organisme non humain est mis en culture avec une substance de test en présence ou en l'absence d'acide mévalonique, et dans lequel une substance de test, qui n'inhibe pas la croissance de l'organisme non humain en présence d'acide mévalonique mais qui inhibe la croissance de l'organisme non humain en l'absence d'acide mévalonique, indique que ladite substance de test inhibe spécifiquement la voie non mévalonate, et ladite substance de test est sélectionnée en tant que substance candidat.

2. Procédé selon la revendication 1, dans lequel l'ADN codant pour les enzymes nécessaires pour utiliser la voie mévalonate en présence d'acide mévalonique est l'un quelconque des ADN suivants:
(1) un ADN ayant toutes les séquences nucléotidiques des SEQ ID N° 9 à 12 ;
(2) un ADN qui comporte une délétion, une substitution, un ajout et/ou une insertion d'un ou de plusieurs nucléotides dans l'ADN de (1) ci-dessus, et qui peut permettre à la voie mévalonate de fonctionner en présence d'acide mévalonique ; ou
(3) un ADN qui peut s'hybrider avec l'ADN de (1) dans des conditions stringentes, et qui peut permettre à la voie mévalonate de fonctionner en présence d'acide mévalonique.

3. Procédé pour cribler des substances qui inhibent spécifiquement la voie non mévalonate en utilisant un organisme non humain capable d'utiliser à la fois la voie mévalonate et la voie non mévalonate en tant que voies biosynthétiques de l'isopentényl diphosphate (IPP) et du diméthylallyl diphosphate (DMAPP), où l'organisme non humain capable d'utiliser à la fois la voie mévalonate et la voie non mévalonate est un organisme non humain capable d'utiliser la voie mévalonate en l'absence d'acide mévalonique, et où ledit organisme non humain est un transformant obtenu par l'introduction d'un ADN codant pour toutes les enzymes nécessaires pour utiliser la voie mévalonate en présence d'acide mévalonique, à l'intérieur d'un organisme non humain possédant la voie non mévalonate, où lesdites enzymes sont au moins HMG-CoA synthase, HMG-CoA réductase, phosphomévalonate kinase, diphosphomévalonate décarboxylase, mévalonate kinase et IPP isomérase, et dans lequel l'organisme non humain capable d'utiliser à la fois la voie mévalonate et la voie non mévalonate ainsi qu'un organisme non humain qui est incapable d'utiliser la voie mévalonate mais qui est capable d'utiliser la voie non mévalonate, sont chacun mis en culture avec une substance de test, et où une substance de test qui n'inhibe pas la croissance de l'organisme non humain qui est capable d'utiliser à la fois la voie mévalonate et la voie non mévalonate mais qui inhibe la croissance de l'organisme non humain qui est incapable d'utiliser la voie mévalonate mais qui est capable d'utiliser la voie non mévalonate, indique que ladite substance de test inhibe spécifiquement la voie non mévalonate, et ladite substance de test est sélectionnée en tant que substance candidat.

4. Procédé selon la revendication 3, dans lequel l'ADN codant pour les enzymes nécessaires pour utiliser la voie mévalonate en l'absence d'acide mévalonique est l'un quelconque des ADN suivants :
(1) un ADN ayant une séquence nucléotidique de SEQ ID N° 13 ;
(2) un ADN qui comporte une délétion, une substitution, un ajout et/ou une insertion d'un ou de plusieurs nucléotides de la séquence nucléotidique de SEQ ID N° 13, et peut permettre à la voie mévalonate de fonctionner en l'absence d'acide mévalonique ; ou
(3) un ADN qui peut s'hybrider avec l'ADN de (1) dans des conditions stringentes, et qui peut permettre à la voie mévalonate de fonctionner en l'absence d'acide mévalonique.

5. Procédé selon l'une quelconque des revendications 1 à 4, dans lequel l'organisme non humain n'ayant pas d'IPP isomérase est utilisé en tant que ledit organisme non humain.

6. Procédé selon l'une quelconque des revendications 1 à 5, dans lequel l'organisme est une bactérie.

7. Procédé selon l'une quelconque des revendications 1 à 6, dans lequel l'organisme est une bactérie à Gram positif.

8. Procédé selon l'une quelconque des revendications 1 à 7, dans lequel l'organisme est *Bacillus subtilis.*

9. Procédé selon l'une quelconque des revendications 1 à 8 qui est utilisé pour le criblage de composés candidats pour des agents antibactériens, des herbicides ou des agents antimalariques.

10. Transformant non humain qui est obtenu par l'introduction d'un ADN codant pour toutes les enzymes nécessaires pour utiliser la voie mévalonate en tant que voie biosynthétique du diphosphate d'isopentényle (IPP) et du diméthylallyl diphosphate (DMAPP) en présence d'acide mévalonique, à l'intérieur d'un organisme non humain possédant uniquement la voie non mévalonique, dans lequel lesdites enzymes sont au moins phosphomévalonate kinase, diphosphomévalonate décarboxylase, mévalonate kinase et IPP isomérase.

11. Transformant selon la revendication 10, dans lequel l'ADN codant pour les enzymes nécessaires pour utiliser la voie mévalonate en présence d'acide mévalonique est l'un quelconque des ADN suivants :
(1) un ADN ayant toutes les séquences nucléotidiques des SEQ ID N° 9 à 12 ;
(2) un ADN qui comporte une délétion, une substitution, un ajout et/ou une insertion d'un ou de plusieurs nucléotides dans l'ADN de (1) ci-dessus, et qui peut permettre à la voie mévalonate de fonctionner en présence d'acide mévalonique ; ou
(3) un ADN qui peut s'hybrider avec l'ADN de (1) dans des conditions stringentes, et qui peut permettre à la voie mévalonate de fonctionner en présence d'acide mévalonique.
